# EUROPEAN PATENT APPLICATION

(11) **EP 1 661 582 A1**
(43) Date of publication of application: **31.05.2006**
(21) Application number: 04772286.3
(22) Date of filing: 20.08.2004
(51) Int. Cl.: A61K 45/00, A61K 39/395, A61P 35/00

(54) **CANCER METASTASIS INHIBITOR**

(30) Priority: 21.08.2003 JP 2003297871; 10.05.2004 JP 2004139707
(71) Applicant: KYOWA HAKKO KOGYO CO., LTD., Chiyoda-ku, Tokyo 100-8185 (JP); JAPAN as represented by PRESIDENT OF NATIONAL CANCER CENTER, Chuo-ku, Tokyo 104-0045 (JP)
(72) Inventor: OCHIAI, Atsushi, Chiba 277-0882 (JP); SHITARA, Kenya; c/o BioFrontier Laboratories, ome, Machida-shi, Tokyo;194-8533 (JP); NAKAMURA, Kazuyasu; c/oBioFrontier Laboratories, 3-chome, Machida-shi, Tokyo;194-8533 (JP); OHKI, Yuji; c/o Tokyo Research Laboratories, Kyowa, 3-chome,Machida-shi, Tokyo;194-8533 (JP)
(74) Representative: Vossius & Partner
(86) International application number: PCT/JP2004/012330
(87) International publication number: WO 2005/018671

(57) **Abstract**

An object of the present invention is to provide a cancer metastasis inhibitor or, more particularly, a useful pharmaceutical composition for treating of bone metastasis and liver metastasis.

In accordance with the invention, a cancer metastasis inhibitor comprising a substance inhibiting the activity of IGF (insulin-like growth factor)-I and IGF-II is an active ingredient is provided. The inhibitor can significantly suppress the metastasis of cancer.

SEQUENCE LISTING

<110> KYOWA HAKKO KOGYO CO., LTD.

<110> JAPAN AS REPRESENTED BY PRESIDENT OF NATIONAL CANCER CENTER

<120> Cancer metastasis inhibitor

<130> PH-2217-PCT

<150> JP2003-297871

<151> 2003-08-21

<150> JP2004-139707

<151> 2004-05-10

<160> 10

<170> PatentIn Ver. 2.0

<210> 1

<211> 18

<212> PRT

<213> Homo sapiens

<400> 1

Gly Pro Glu Thr Leu Ser Gly Ala Glu Leu Val Asp Ala Leu Gln Phe
  1               5                  10                  15

Val Cys

<210> 2

<211> 18

<212> PRT

<213> Homo sapiens

<400> 2

Cys Leu Gln Phe Val Ala Gly Asp Arg Gly Phe Tyr Phe Asn Lys Pro

1               5                   10                  15

Thr Gly

<210> 3

<211> 13

<212> PRT

<213> Homo sapiens

<400> 3

Cys Tyr Phe Asn Lys Pro Thr Gly Tyr Gly Ser Ser Ser

1               5                   10

<210> 4

<211> 14

<212> PRT

<213> Homo sapiens

<400> 4

Cys Thr Gly Tyr Gly Ser Ser Ser Arg Arg Ala Pro Gln Thr

1                   5                       10

<210> 5

<211> 12

<212> PRT

<213> Homo sapiens

<400> 5

Arg Arg Ala Pro Gln Thr Gly Ile Val Asp Glu Cys

1                   5                       10

<210> 6

<211> 17

<212> PRT

<213> Homo sapiens

<400> 6

Cys Thr Gly Ile Val Asp Glu Ala Ala Phe Arg Ser Ala Asp Leu Arg

1                   5                       10                      15

Arg

<210> 7

<211> 19

<212> PRT

<213> Homo sapiens

<400> 7

Cys Asp Leu Arg Arg Leu Glu Met Tyr Ala Ala Pro Leu Lys Pro Ala
1               5                   10                  15

Lys Ser Ala

<210> 8
<211> 9
<212> PRT
<213> Homo sapiens

<400> 8
Asp Leu Arg Arg Leu Glu Met Tyr Cys
1               5

<210> 9
<211> 10
<212> PRT
<213> Homo sapiens

<400> 9
Cys Ala Pro Leu Lys Pro Ala Lys Ser Ala
1               5                   10

<210> 10
<211> 17
<212> PRT
<213> Homo sapiens

<400> 10

Cys Thr Gly Ile Val Asp Glu Cys Cys Phe Arg Ser Cys Asp Leu Arg

1               5                   10                  15

Arg

## Description

### Technical Field

The present invention relates to a cancer metastasis inhibitor comprising a substance which inhibits the activity of insulin-like growth factor (hereinafter, referred to as IGF) I and IGF-II as an active ingredient.

### Background ,Art

Metastasis of cancer is a phenomenon where cancer cells spread in a body and are reproliferated. The worst factor for the death of a patient is metastasis of cancer. Cancer which remains in the primary focus can be removed by a surgical operation relatively easily. However, it is difficult to remove cancer which has metastasized to various focus by a surgical operation (*Nikkei Science*, December 1996, page 30).

Metastasis of cancer is apt to be generated in specific organs. As well as lung and liver, bone is a major target organ for distant metastasis of cancer. Especially in patients suffering from prostate cancer, breast cancer, lung cancer and multiple myeloma, bone metastasis of cancer occurs at a high frequency. For cancer which has metastasized to bone, the conventional treating methods for cancer are rarely effective and a patient is tormented by significant physical pain and mental anxiety whereby QOL (quality of life) is markedly damaged (*Nature Reviews Cancer*, 2, 584-593, 2001; *Jikken Igaku*, 16, 149-155, 1998). Accordingly, there has been a demand for a useful treating method for treating metastasis to bone. In addition, metastasis to liver occurs at a high frequency in cancer of digestive organs, particularly in advanced colorectal cancer and, therefore, metastasis to liver is a major factor for poor prognosis of colorectal cancer. Accordingly, there has been a demand for a useful treating method for treating liver metastasis.

With regard to mechanism of bone metastasis of cancer, there are still many ambiguous points as well. As one of important mechanisms therefor, there has been pointed out a possibility that various growth factors abundantly contained in bone promote the bone metastasis of cancer cell. Growth factor stored in bone and the stored amounts thereof (ng/g of dry bone weight) are as follows [IGF-II: 1,300-1,700 ng/g; transforming β growth factor (TGFβ): 400-460 ng/g; IGF-I: 85-170 ng/g; platelet-derived growth factor (PDGF): 50-70 ng/g; basic fibroblast growth factor (bFGF): 40-80 ng/g; acidic fibroblast growth factor (aFGF) : 0.5-12 ng/g; bone morphogenic protein (BMP): 1-2 ng/g; activin (1 ng/g) (*Jikken Igaku*, 16, 149-155, 1998)]. Cancer cell *per se* can not destroy the bone. However, there has been pointed out a possibility that, after bone is destroyed by osteoclast, the above-mentioned growth factor stored in bone are released to promote proliferation of cancer cell.

With regard to mechanism of liver metastasis of cancer, there are still many ambiguous points as well. In the case of hematogenous metastasis of digestive organ's cancer, because of anatomical reasons that cancer cell released from primary focus into blood stream firstly reach the liver and then are caught by microvessels, possibility that digestive organ's cancer has a tendency to metastasize to the liver.

There has been also a report for the relationship between IGF and metastasis of cancer. It has been reported that, when a dominant negative IGF-I receptor (hereinafter, referred to as IGF-IR) is expressed in a human colorectal cancer cell line, metastases to liver is suppressed in mice (*Laboratory Investigation,* 82, 1377-1389, 2002). It has been also reported that, when dominant negative IGF-IR is expressed in human breast cancer cell line, metastases to lung, liver and lymph node are suppressed in mice (*Cancer Research*, 58, 3353-3361, 1998). H-59 which is a sub-strain of Lewis lung cancer is a tumor cell line showing a high frequency of metastatic property to liver and it has been reported that, as the result of analyzing a factor which promotes the proliferation of H-59 contained in culture supernatant of hepatic cell, IGF-I shows a promoting activity for the proliferation of H-59. It has been reported that since M-27 which is another sub-strain of Lewis lung cancer metastasizing only to lung has no promoting activity for the proliferation of IGF-I, IGF-I participates in liver metastasis of cancer cell (*Cancer Research*, 54, 3732-3737, 1994).

In fact, there have been reports on anti-tumor effects by suppression of IGF activity (*Biochimica et Biophysica Acta*, 1332, F105-F126, 1997) such as a case, when antisense RNA to IGF-IR is expressed, tumor-forming property and metastatic property to lung of highly metastatic human breast cancer cell are lowered in mice whereby elongation of life time is noted (*Cancer Gene Therapy*, 7, 384-395, 2000), that proliferation of human striated muscle sarcoma cell and human breast cancer cell grafted to mice is suppressed by an anti-IGF-IR antibody (*Cancer Research,* 54, 5531-5534, 1994; *Journal of Clinical Investigation,* 84, 1418-1423, 1989; *Breast Cancer Research & Treatment,* 22, 101-106, 1992). On the other hand, it has been shown that, although an anti-IGF-IR antibody suppresses the engraftment of human breast cancer cell showing estrogen-independent growth grafted to mice, it can not suppress engraftment of human breast cancer cell showing estrogen-dependent growth or growth of the engrafted human breast cancer cell to thereby suggest that a mere suppressive effect of IGF-IR does not achieve a sufficient anti-tumor effect (*Breast Cancer Research & Treatment,* 22, 101-106, 1992).

With regard to antibody to IGF or, in other words, anti-IGF antibody, some antibodies have been known already. As to a typical antibody to human IGF-I (anti-hIGF-I antibody), sm1.2 has been reported (*Proceedings of the National Academy of Sciences of the United States of America*, 81, 2389-2392, 1984). It has been revealed that sm1.2 has a crossreactivity to hIGF-II of about 40%, can detect 100 ng of hIGF-I by a western blotting method at a concentration of 1 to 2 µg/ml and inhibits the proliferation of mouse fibroblast cell line BALB/c3T3 by 20 ng/mL of hIGF-I at a concentration of 10 to 30 µg/ml (*Proceedings of the National Academy of Sciences of the United States of America*, 81, 2389-2392, 1984; *Journal of Clinical Investigation*, 99, 2961-2970, 1997).

Besides the above, Val⁵⁹-SmC121 has been known as an anti-hIGF-I antibody and it has been reported that the antibody does not react with human insulin and hIGF-II, recognizes a peptide containing the 10th to 12th Leu-Val-Asp of hIGF and shows 1 ng/mL of hIGF-I detection sensitivity in a radioimmunoassay using ¹²⁵I-hIGF-I (*Journal of Endocrinology*, 125, 327-335, 1990). 41/81 has a reactivity of 3% to hIGF-II and, in a radioimmunoassay using ¹²⁵I-hIGF-I, it shows 1 ng/mL of hIGF-I detection sensitivity (*FEBS Letters*, 149, 109-112, 1982). 35I17 has been reported to have a crossreactivity to hIGF-II of about 0.5%, to be able to detect 1 µg of hIGF-I by a western blotting method at a concentration of 1 µg/mL, to completely inhibit the proliferation of mouse fibroblast cell line BALB/c3T3 by hIGF-I in a concentration of not lower than 12 µg/mL, to inhibit a self-phosphorylation of hIGF-IR by 1 µg/mL of hIGF-I at a concentration of 30 µg/mL and to show 0.1 nM of hIGF-I detection sensitivity in a radioimmunoassay using ¹²⁵I-hIGF-I (*Hybridoma*, 16, 513-518, 1997). It has been reported that BPL-M23 shows a binding activity of 10.5 liters/nmol to hIGF-I while, to hIGF-II and human insulin, it shows crossreactivity of 0.8% and 0.0001%, respectively, that, although it shows reactivity to IGF of goat, pig, sheep, cattle and rabbit, it does not react with IGF of rat and mouse and that it suppresses a fat formation by hIGF-I to fat cells of rat (*Journal of Molecular Endocrinology*, 2, 201-206, 1989). 7A1, 1B3, 4C1 and 5A7 have been reported to recognize different epitopes of C and D domains of hIGF-I and to show crossreactivity to hIGF-II of 6.6%, 0.83%, 12% and 1.2%, respectively (*Hybridoma*, 12, 737-744, 1993). 3DI/2/1 has been reported that, although it shows reactivity with IGF-I of human and guinea pig, it does not react with IGF-I of rabbit, rat and mouse and that it shows crossreactivity of 7% with hIGF-II (*Journal of Clinical and Metabolism*, 54, 474-476, 1982).

With regard to a typical antibody to human IGF-II (anti-hIGF-II antibody), S1F2 has been reported. It has been revealed that S1F2 has crossreactivity of about 10% with hIGF-I, that it can detect 10 to 100 ng of hIGF-II by a western blotting method at a concentration of 1 µg/mL and that it inhibits the promoting action for the DNA synthesis of human fibroblast cells by 100 ng/mL of hIGF-II at a concentration of 100 µg/mL (*Diabetes Research and Clinical Practice*, 7, S21-S27, 1989; *Endocrinology*, 124, 870-877, 1989). It has been reported that 2H11, 2B11, ID5 and ID9 react with hIGF-II, do not react with hIGF-I and can determine 1 ng/mL of hIGF-II by a competitive enzyme immunoassay (hereinafter, referred to as ELISA) (Japanese published unexamined application No. 252987/93). However, those antibodies have not been known to be useful in inhibition of metastasis of cancer.

Accordingly, an object of the present invention is to provide a cancer metastasis inhibitor and, more particularly, to provide a pharmaceutical composition which is useful for suppressing bone and liver metastasis of cancer.

### Disclosure of the Invention

Thus, the present invention includes the following inventions (1) to (4).
(1) A cancer metastasis inhibitor comprising a substance which inhibits the activity of insulin-like growth factor-I (IGF-I) and insulin-like growth factor-II (IGF-II) as an active ingredient.
(2) The inhibitor according to (1), wherein the substance is selected from the group consisting of the following (a) to (e).
   (a) an antibody or an antibody fragment which specifically binds to IGF-I and IGF-II and inhibits the activity of IGF-I and IGF-II;
   (b) a composition comprising an antibody or an antibody fragment which specifically binds to IGF-I and inhibits the activity of IGF-I and an antibody or an antibody fragment which specifically binds to IGF-II and inhibits the activity of IGF-II;
   (c) a composition comprising combination of a composition comprising an antibody or an antibody fragment which specifically binds to IGF-I and inhibits the activity of IGF-I and a composition comprising an antibody or an antibody fragment which specifically binds to IGF-II and inhibits the activity of IGF-II;
   (d) a conjugate of an antibody or an antibody fragment which specifically binds to IGF-I and inhibits the activity of IGF-I with an antibody or an antibody fragment which specifically binds to IGF-II and inhibits the activity of IGF-II; and
   (e) a conjugate of any of the above (a) to (d) with other molecules.
(3) The inhibitor according to (2), wherein the antibody is a monoclonal antibody.
(4) The inhibitor according to (2) or (3), wherein the antibody fragment is an antibody fragment selected from the group consisting of Fab, Fab' , F(ab')₂, single-chain antibody (scFv), dimerized variable region (diabody), disulfide-stabilized variable region (dsFv) and CDR-containing peptide.

### Brief Description of the Drawings

Fig. 1 shows a growth inhibitory activity of an antibody KM 1468 for the proliferation of human prostate cancer cell MDA PCa 2b. The abscissa shows types and concentration of the samples added and the ordinate shows proliferation of the cell.
Fig. 2A shows an inhibitory activity of an antibody KM 1468 to bone metastasis of prostate cancer in an early metastasis model and Fig. 2C shows an inhibitory activity of an antibody KM 1468 to bone metastasis of prostate cancer in a late metastasis model. Fig. 2B shows PSA concentrations in serum upon addition and non-addition of an antibody KM 1468 in an early metastasis model and Fig. 2D shows PSA concentrations in serum upon addition and non-addition of an antibody KM 1468 in a late metastasis model.
Fig. 3 shows the result of detection by a western blotting for phosphorylation of IGF-IR, Akt and MAPK stimulated with hIGF-I and for suppression of phosphorylation by an antibody KM 1468 and an antibody KM 1762 using human multiple myeloma cell RPMI8226.
Fig. 4 shows inhibitory activity for the proliferation by an antibody KM 1468 (1 µg/mL) to the proliferation of human multiple myeloma cell RPMI8226 stimulated with hIGF-1.
Fig. 5 shows inhibitory activity for the proliferation by an antibody KM 1468 of various concentrations to the proliferation of human multiple myeloma cell RPMI8226 stimulated with hIGF-I.
Fig. 6 shows inhibitory activity to bone metastasis of multiple myeloma by an antibody KM 1468 in an early metastasis model.
Fig. 7 shows inhibitory activity to bone metastasis of breast cancer by an antibody KM 1468 in an early metastasis model.
Fig. 8 shows inhibitory activity to liver metastasis of colorectal cancer cell line when an anti-IGF antibody is administered in a liver metastasis model of colorectal cancer. A horizontal line in each column shows quartiles and, from top to bottom, the lines show 90th, 75th, 50th, 25th and 10th percentiles. In each column, the signs "open-circular" show maximum and minimum values in each group.
Fig. 9 shows AI (apoptosis index) values in tumor slices when an anti-IGF antibody is administered in a liver metastasis model of colorectal cancer. A horizontal line in each column shows a quartiles and, from top to bottom, the lines show 90th, 75 th, 50 th, 25 th and 10th percentiles. In each column, the signs "open-circular" show maximum and minimum values in each group.

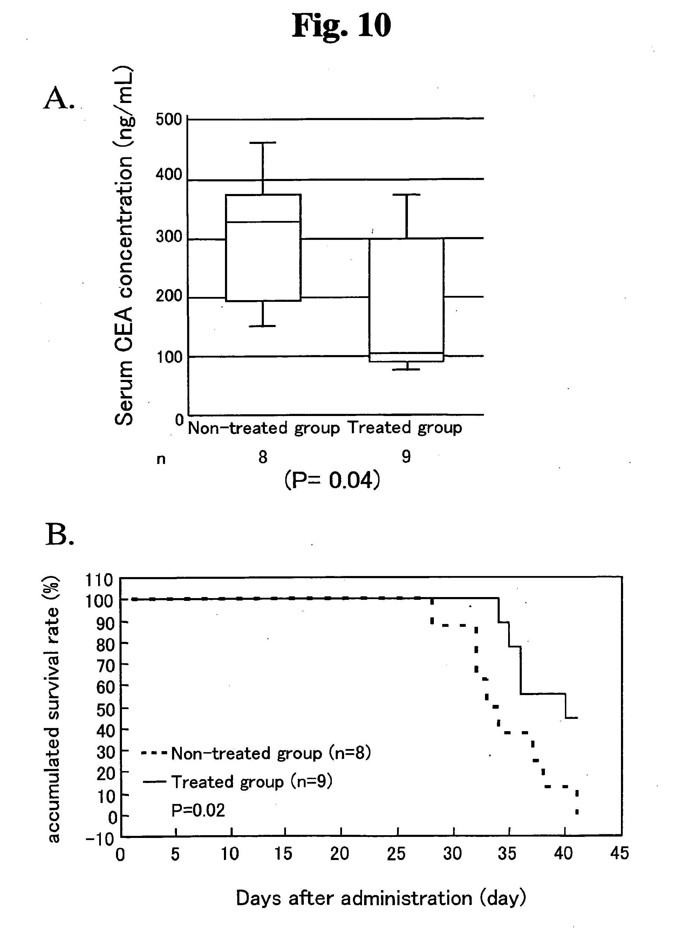

Fig. 10A shows the therapeutic effect for liver metastatic focus of colorectal cancer cell line upon administration of the anti-hIGF-I in a liver metastatic model of colorectal cancer. Horizontal lines in each column show quartiles and, from top to bottom, they show 90th, 75th, 50th, 25th and 10th percentiles, respectively. Fig. 10B shows the macrobiotic effect upon administration of the anti-hIGF-I in a liver metastatic model of colorectal cancer. The abscissa shows days elapsed from initiation of the administration and the ordinate shows an accumulated survival rate. A solid line shows a treated group while a broken line shows a non-treated group.
Fig. 11 shows the specific reactivity of a monoclonal antibody to hIGF-I (binding ELISA). The abscissa shows combination of antibody with antigen while the ordinate shows binding activity (OD415).
Fig. 12 shows reactivity of a monoclonal antibody to hIGF-I having an authentic three-dimensional structure in a liquid phase system (competitive ELISA). The abscissa shows concentration of the hIGF-I added while the ordinate shows binding activity (OD415).
Fig. 13 shows the reactivity of the antibody KM 1468 and sm1.2 to hIGF-I. The abscissa shows concentration of the antibody (µg/mL) while the ordinate shows binding activity (OD415). Open-circular and open-square show reactivity of KM 1468 and that of sm1.2, respectively.
Fig. 14 shows inhibitory activities of various factors to the binding of the antibody KM1468 and sm1.2 to hIGF-I. The abscissa shows concentration of each factor while the ordinate shows binding activity (%). A, B, C and D show activities by hIGF-I, hIGF-II, human insulin and mIGF-I, respectively. Open-circular and open-square show reactivity of KM 1468 and that of sm1.2, respectively.

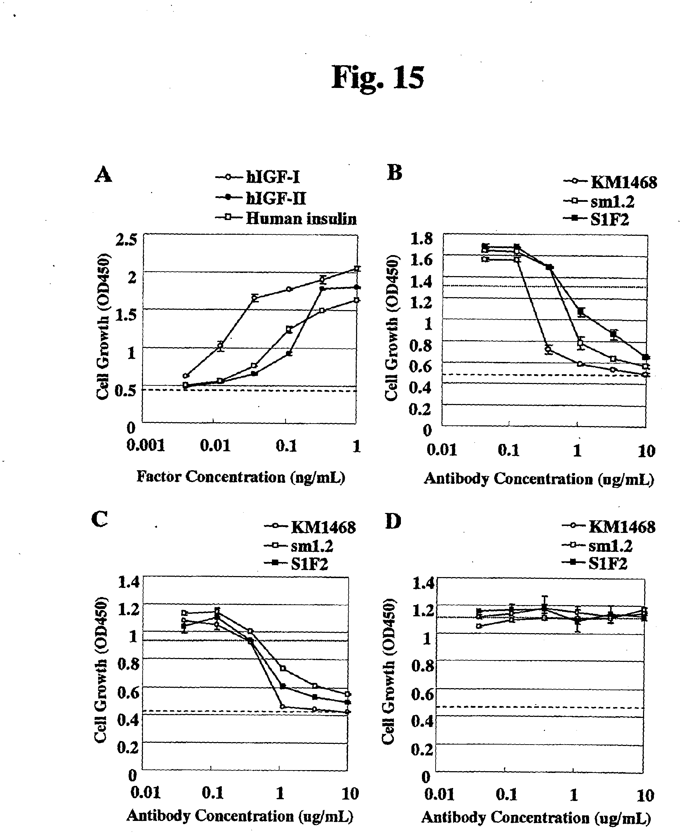

Fig. 15 shows influences of the antibodies KM 1468, sm1.2 and S1F2 on the proliferation of a human breast cancer cell line MCF7 by hIGF and human insulin. "A" shows a cell proliferation activity by each factor. The abscissa shows concentration of each factor (µg/mL) while the ordinate shows the proliferation (OD450). Open-circular, closed-circular and open-square show activities of hIGF-I, hIGF-II and human insulin, respectively. B, C and D show influences of various antibodies to proliferation activity by hIGF-I, hIGF-II and human insulin, respectively. The abscissa shows concentration of antibody (µg/mL) while the ordinate shows the proliferation (OD450). A dotted line shows proliferation in the absence of antibody while a broken line shows proliferation in the absence of each factor. Open-circular, open-square and closed-square show activities of KM 1468, sm1.2 and S1F2, respectively.

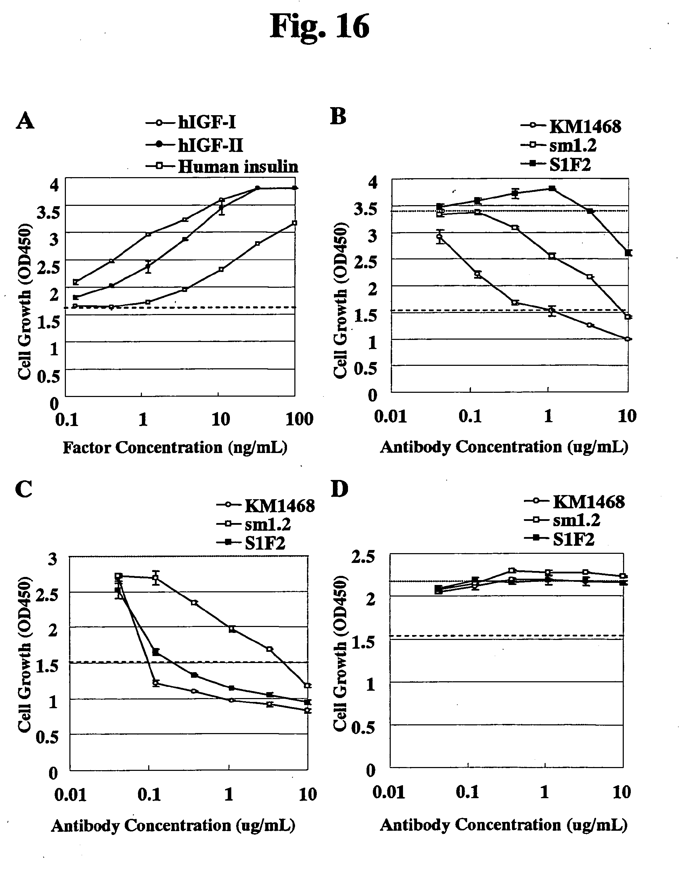

Fig. 16 shows influences of the antibodies KM 1468, sm1.2 and S1F2 on the proliferation of a human colorectal cancer cell line HT-29 by hIGF and human insulin. "A" shows a cell proliferation activity by each factor. The abscissa shows concentration of each factor (ng/mL) while the ordinate shows the proliferation (OD450). Open-circular, closed-circular and open -square show activities of hIGF-I, hIGF-II and human insulin, respectively. B, C and D show influences of various antibodies to proliferation activity by hIGF-I, hIGF-II and human insulin, respectively. The abscissa shows concentration of antibody (µg/mL) while the ordinate shows the proliferation (OD450). A dotted line shows proliferation in the absence of antibody while a broken line shows proliferation in the absence of each factor. Open-circular, open-square and closed-square show activities of KM 1468, sm1.2 and S1F2, respectively.

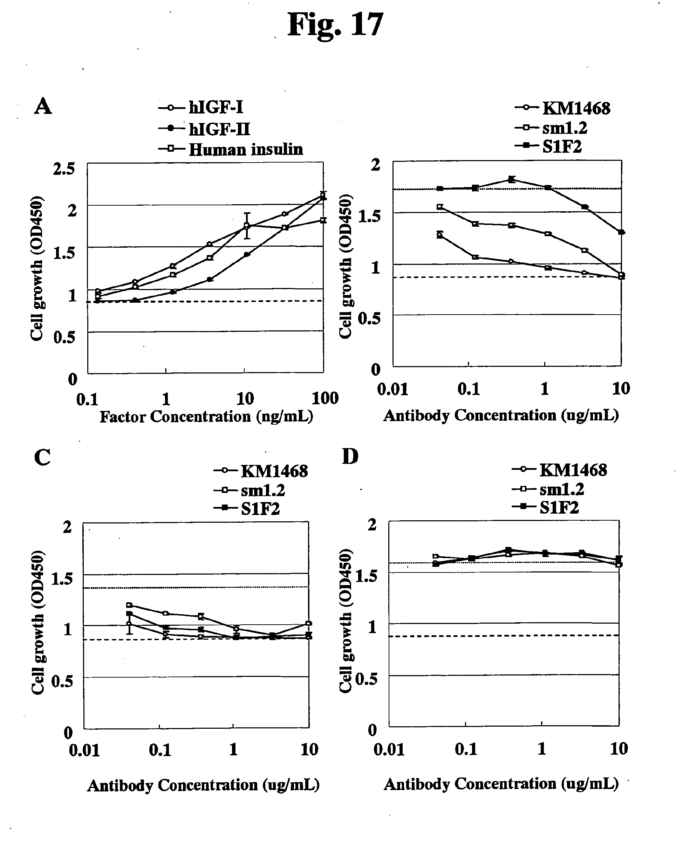

Fig. 17 shows influences of the antibodies KM 1468, sm1.2 and SlF2 on the proliferation of a human osteosarcoma cell line MG63 by hIGF and human insulin. "A" shows a cell proliferation activity by each factor. The abscissa shows concentration of each factor (ng/mL) while the ordinate shows the proliferation (OD450). Open-circular, closed-circular and open -square show activities of hIGF-I, hIGF-II and human insulin, respectively. B, C and D show influences of various antibodies to proliferation activity by hIGF-I, hIGF-II and human insulin, respectively. The abscissa shows concentration of antibody (µg/mL) while the ordinate shows the proliferation (OD450). A dotted line shows proliferation in the absence of antibody while a broken line shows proliferation in the absence of each factor. Open-circular, open-square and closed-square show activities of KM 1468, sm1.2 and S1F2, respectively.
Fig. 18 shows inhibitory activities by various peptides in binding of an antibody KM 1468 to hIGF-I . The abscissa shows concentration of various peptide (µg/mL) while the ordinate shows binding activity (%). The various peptides used are mentioned in the drawings.

The invention will now be illustrated in detail as hereunder. The present application claims priorities on Japanese Patent Application No. 2003-297871 filed on August 21, 2003 and Japanese Patent Application No. 2004-139707 filed on May 10, 2004 and includes the contents mentioned in the specifications and/or drawings of those patent applications.

### A. Active ingredient of the cancer metastasis inhibitor of the present invention

The active ingredient of the cancer metastasis inhibitor of the present invention may be any substance so long as it is a substance which inhibits the activities of IGF-I and IGF-II.

The substance which inhibits the activities of IGF-I and IGF-II may be a single substance or may be a composition comprising plural substances and, in the case of a composition comprising plural substances, each of the substances may be used either at the same time or separately.

Examples of the substance which inhibits the activities of IGF-I and IGF-II are
(a) an antibody or an antibody fragment which specifically binds to IGF-I and IGF-II and inhibits the activity of IGF-I and IGF-II;
(b) a composition comprising an antibody or an antibody fragment which specifically binds to IGF-I and inhibits the activity of IGF-I and an antibody or an antibody fragment which specifically binds to IGF-II and inhibits the activity of IGF-II;
(c) a composition comprising combination of a composition comprising an antibody or an antibody fragment which specifically binds to IGF-I and inhibits the activity of IGF-I and a composition comprising an antibody or an antibody fragment which specifically binds to IGF-II and inhibits the activity of IGF-II;
(d) a conjugate of an antibody or an antibody fragment which specifically binds to IGF-I and inhibits the activity of IGF-I with an antibody or an antibody fragment which specifically binds to IGF-II and inhibits the activity of IGF-II; and
(e) a conjugate of any of the above (a) to (d) with other molecules.

The aforementioned expression of "to inhibit the activity of IGF-I and IGF-II" means that any of the activities of IGF-I and IGF-II is inhibited and its specific example is to inhibit the activity of promoting the cell proliferation by IGF-I and IGF-II.

The expression of "an antibody or an antibody fragment which specifically bonds to IGF-I and IGF-II and inhibits the activity of IGF-I and IGF-II" used in the present invention means an antibody or an antibody fragment which specifically binds to both IGF-I and IGF-II and inhibits the activity of both IGF-I and IGF-II. Specific examples thereof are an antibody or an antibody fragment which recognizes the epitope existing in an authentic IGF-I and an authentic IGF-II, an antibody or an antibody fragment which recognizes the three-dimensional structure of IGF-I and IGF-II, and the like.

Although the aforementioned antibody or the antibody fragment used in the present invention may be any of a polyclonal antibody and a monoclonal antibody, a monoclonal antibody is preferred.

The aforementioned antibody or the antibody fragment also includes "an antibody of the non-human animal", "a recombinant antibody", antibody fragments thereof, and the like.

Examples of the "recombinant antibody" are a humanized antibody, a human antibody, and the like and examples of the "humanized antibody" are a human chimeric antibody and a human CDR-grafted antibody.

The "human chimeric antibody" means an antibody comprising VH and VL of an antibody of the non-human animal and CH and CL of a human antibody. As CH of the human chimeric antibody, although any CH may be used so long as it belongs to human immunoglobulin (hereinafter, referred to as hIg), that of an hIgG class is preferred and any of subclasses hIgG1, hIgG2, hIgG3 and hIgG4 belonging to an hIgG class may be used as well. With regard to CL of a human chimeric antibody, any CL may be used so long as it belongs to hIg and any of a κ class and a λ class may be used.

The non-human animals are mouse, rat, hamster, rabbit and the like.

The "human CDR-grafted antibody" means an antibody where CDRs of VH and VL of an antibody of the non-human animal are grafted to an appropriate positions of VH and VL of a human antibody.

The human CDR-grafted antibody according to the present invention can be produced by designing and constructing cDNAs coding for a V region in which CDRs of VH and VL of the non-human animal is ligated with a framework (hereinafter, referred to as FR) of VH and VL of any human antibody, inserting them respectively into expression vector for animal cell having cDNAs coding for CH and CL of a human antibody to construct a human CDR-grafted antibody expression vector followed by introducing the expression vector into animal cell for expression.

With regard to CH for a human CDR-grafted antibody, although any CH may be used so long as it belong to hIg, that of an hIgG class is preferred and any of subclasses hIgG1, hIgG2, hIgG3 and hIgG4 belonging to an hIgG class may be used as well. With regard to CL of a human CDR-grafted antibody, any CL may be used so long as it belongs to hIg and that of a κ class and a λ class may be used.

Though the "human antibody" generally means an antibody naturally existing in human body but it also includes an antibody which is prepared from a human antibody phage library and from human antibody-producing transgenic animals prepared by the recent progresses in techniques in genetic engineering, cell engineering and developmental engineering. Regarding the antibody existed in the human body, for example, a lymphocyte capable of producing said antibody can be cultured by isolating a human peripheral lymphocyte, immortalizing by infecting with EB virus or the like and then cloning, and said antibody can be purified from the culture supernatant. The human antibody phage library is a library in which antibody fragments of Fab, scFv and the like are expressed on the phage surface by inserting an antibody gene prepared from human B cell into a phage gene. A phage expressing antibody fragments having desired antigen binding activity on the surface can be recovered from said library using the binding activity to an antigen-immobilized substrate as an index. Said antibody fragments can be further converted into a human antibody molecule comprising two full length H chains and two full length L chains by genetic engineering techniques. The human antibody-producing transgenic animal means an animal in which a human antibody gene is integrated into its cells. For example, a human antibody-producing transgenic mouse can be prepared by introducing a human antibody gene into a mouse ES cell, transplanting said ES cell into early embryo of a mouse and then developing. Regarding the method for preparing a human antibody from a human antibody-producing transgenic animal, the human antibody can be produced and accumulated in a culture supernatant by culturing a human antibody-producing hybridoma obtained by a hybridoma preparation method generally carried out in non-human animal.

The antibody or the antibody fragment which is preferably used in the present invention includes a monoclonal antibody KM 1468 produced by hybridoma KM 1468 (FERM BP-7978),an anti-hIGF-I monoclonal antibody sm1.2 (Upstate Biology) which reacts with hIGF-I and also have about 40% crossreactivity with hIGF-II, or the like.

With regard to "a composition comprising the antibody or the antibody fragment which specifically binds to IGF-I and inhibits the activity of IGF-I and the antibody or the antibody fragment which specifically binds to IGF-II and inhibits the activity of IGF-II", any composition may be used so long as it is a composition comprising the antibody or the antibody fragment which inhibits the activity of IGF-I and the antibody or the antibody fragment which inhibits the activity of IGF-II.

The expression of "the antibody or the antibody fragment which specifically binds to IGF-I and inhibits the activity of IGF-I" means an antibody which specifically binds to IGF-I but does not specifically bind to IGF-II (having no crossreactivity) and its examples are AF791 (manufactured by R & D) which is an antibody to mouse IGF-I (hereinafter, referred to as mIGF-I), 56408 (manufactured by R & D) which is a monoclonal antibody to human IGF-I (hereinafter, mentioned as hIGF-I) and M23/ILG1-001 (manufactured by Biogenesis).

The expression of "the antibody or the antibody fragment which specifically binds to IGF-II and inhibits the activity of IGF-II" means an antibody which specifically binds to IGF-II but does not specifically bind to IGF-I (having no crossreactivity) and its examples are AF792 (manufactured by R & D) which is an antibody to mIGF-II and S1F2 (manufactured by Upstate Biology) which is a monoclonal antibody to hIGF-II.

The expression of "a composition comprising combination of a composition comprising an antibody or an antibody fragment which specifically binds to IGF-I and inhibits the activity of IGF-I and a composition comprising an antibody or an antibody fragment which specifically binds to IGF-II and inhibits the activity of IGF-II" is a composition in which an agent comprising "the antibody or the antibody fragment which specifically binds to IGF-I and inhibits the activity of IGF-I" and an agent comprising "the antibody or the antibody fragment which specifically binds to IGF-II and inhibits the activity of IGF-II" are separately prepared and those agents are combined for a simultaneous use or a successive use.

With regard to "a conjugate of an antibody or an antibody fragment which specifically binds to IGF-I and inhibits the activity of IGF-I with an antibody or an antibody fragment which specifically binds to IGF-II and inhibits the activity of IGF-II", any conjugate may be used so long as it is a conjugate prepared by conjugating the antibody or the antibody fragment inhibiting the activity of IGF-I with the antibody or the antibody fragment inhibiting the activity of IGF-II.

To be more specific, an antibody conjugate where the two kinds of antibodies or antibody fragments are conjugated by the following means may be exemplified.

With regard to a method for conjugating the antibodies, a method where they are chemically bound and a method using protein engineering may be exemplified.

With regard to a method where they are chemically bound, a method where two kinds of antibody molecules are conjugated using a cross-linking agent such as N-succinimidyl-3-(2-pyridyldithiol) propionate and S-acetylmercaptosuccinic acid anhydride may be exemplified.

With regard to a method of conjugating using a protein engineering techniques, any method may be used so long as it is a method where plural antibodies or antibody fragments can be expressed in a conjugate using a protein engineering techniques. Examples of the antibody conjugate prepared by a method of conjugating using a protein engineering techniques are a molecule where two kinds of scFv are linked via an appropriate linker, a molecule where two kinds of antibody Fab' fragments are conjugated via an appropriate linker, an Fc fused protein where two kinds of scFv are conjugated to N-terminal and C-terminal, a heteromolecule of Fc fused protein where two kinds of scFv are conjugated, diabody and an Fc fused protein where diabody is conjugated to N-terminal or C-terminal.

With regard to a conjugate where any of the aforementioned antibodies is conjugated to other molecules, any conjugate may be used so long as it is a conjugate prepared by conjugating any of an antibody or an antibody fragment which specifically binds to IGF-I and inhibits the activity of IGF-I with any of the antibody or the antibody fragment which specifically binds to IGF-II and inhibits the activity of IGF-II; a composition comprising the antibody or the antibody fragment which specifically binds to IGF-I and inhibits the activity of IGF-I and the antibody or the antibody fragment which specifically binds to IGF-II and inhibits the activity of IGF-II; a composition comprising combination of a composition comprising the antibody or the antibody fragment which specifically binds to IGF-I and inhibits the activity of IGF-I and a composition comprising the antibody or the antibody fragment which specifically binds to IGF-II and inhibits the activity of IGF-II; and a conjugate of the antibody or the antibody fragment which specifically binds to IGF-I and inhibits the activity of IGF-I with the antibody or the antibody fragment which specifically binds to IGF-II and inhibits the activity of IGF-II, with at least one kind of other molecules.

Examples of other molecules include radioisotope, agent having low-molecular weight, compound having high-molecular weight and protein. As a method for conjugateing the other molecules such as radioisotope, agent having low-molecular weight, agent having high-molecular weight and protein with the aforementioned antibody, any method may be used and preferred examples thereof are a method where conjugating is carried out chemically ("Introduction to Antibody Technology" by Osamu Kanemitsu, published by Chijin Shoin, 1994), a method where conjugating is carried out using protein engineering techniques, and the like.

An example of a method where conjugating is carried out chemically includes a method where aforementioned other molecules are linked to N-terminal or C-terminal of H chain or L chain of the antibody or the antibody fragment, to an appropriate substituent group or side chain in the antibody or the antibody fragment,to sugar chain in the antibody or the antibody fragment, and the like.

An example of a method of conjugating using a protein engineering techniques includes a method where DNA coding for the antibody or the antibody fragment is ligated with DNA coding for other molecules to be linked such as protein followed by inserting into vector for expression and the expression vector is introduced into an appropriate host cell and expressed whereby a fused product in which the antibody or the antibody fragment is conjugated with other molecules is prepared.

Examples of the radioisotope include ¹³¹I and ¹²⁵I and it can be conjugated with an antibody by, for example, a chloramine T method.

Examples of the agent having low-molecular weight are anti-cancer agents including an alkylating agent such as nitrogen mustard and cyclophosphamide; a metabolism antagonist such as 5-fluorouracil and methotrexate; an antibiotic such as daunomycin, bleomycin, mitomycin C, daunorubicin and doxorubicin; a plant alkaloid such as vincristine, vinblastine and vindesine; and a hormone agent such as tamoxifen and dexamethasone ("Clinical Oncology" edited by Japan Clinical Tumor Study Team, published by Gan to Kagaku Ryohosha, 1996); a steroidal agent such as hydrocortisone and prednisone; a non-steroidal agent such as aspirin and indomethacin; an immunomodulator such as gold thiomalate and penicillamine; an immunosuppressant such as cyclophosphamide and azathioprine; an anti-inflammatory agent including antihistamine agent such as chlorpheniramine maleate and clemastine ("Inflammation and Anti-inflamatory Therapy", published by Ishiyaku Publishers, Inc., 1982) or the like. Examples of a method for conjugating daunomycin with an antibody are a method where daunomycin and amino group of the antibody are linked via glutaraldehyde, a method where an amino group of daunomycin and carboxyl group of the antibody are linked via a water-soluble carbodiimide and the like.

Examples of the compound having high-molecular include polyethylene glycol (hereinafter, mentioned as PEG), albumin, dextran, polyoxyethylene, styrene-maleic acid copolymer, polyvinylpyrrolidone, pyran copolymer, hydroxypropyl methacrylamide and the like. When such a compound having high-molecular weight is conjugated with an antibody or antibody fragment, an effect such as (1) enhancement of stability against various chemical, physical or biological factors, (2) significant extension of blood half-life and (3) disappearance of immunogenicity and suppression of antibody production can be expected ("Bioconjugate Drugs", published by Hirokawa Shoten, 1993). An example of a method for conjugating PEG with an antibody includes a method where an antibody is reacted with a PEG-modifying reagent ("Bioconjugate Drugs", published by Hirokawa Shoten, 1993). Examples of the PEG-modifying reagent include a modifier for e-amino group of lysine (Japanese Published Unexamined Application No. 178926/86), a modifier for carboxyl groups of aspartic acid and glutamic acid (Japanese Published Examined Application No. 23587/81), a modifier for guanidine group of arginine (Japanese Published Unexamined Application No. 117920/90), and the like.

Examples of the protein include cytokines which activate the immunocompetent cells such as human interleukin 2, human granulocyte macrophage colony stimulating factor, human macrophage colony stimulating factor, human interleukin 12 and the like. Toxin such as diphtheria toxin and ricin having an activity of directly damaging the cancer cells can also be used. With regard to a conjugate in which protein is conjugated with an antibody, the conjugate can be produced in such a manner that, for example, cDNA coding for protein is ligated with cDNA coding for an antibody or an antibody fragment to construct a DNA which codes for the conjugate, the DNA is inserted into expression vector for prokaryote or eukaryote and the expression vector is introduced into the prokaryote or the eukaryote to express whereupon the conjugate is produced.

Examples of the antibody fragment used in the present invention include Fab, Fab', F(ab')₂, scFv, diabody, dsFv, CDR-containing peptide, and the like.

Fab is an antibody fragment having a molecular weight of about 50,000 and exhibiting an antigen-binding activity where about one half of N-terminal side of H chain and the full length of L chain, among fragments obtained by treating IgG-type antibody molecule with a protease, papain (cleave an amino acid residue at position 224 of an H chain) are bound together through a disulfide bond.

The Fab which is used in the present invention can be prepared by treating the antibody with a protease, papain. Alternatively, DNA which codes for Fab of the antibody is inserted into expression vector for prokaryote or expression vector for eukaryote and the vector is introduced into prokaryote or eukaryote to express whereupon Fab is produced.

F(ab')₂ is an antibody fragment having a molecular weight of about 100,000 and exhibiting an antigen-binding activity which is slightly larger than the Fab bound via disulfide bond of the hinge region, among fragments obtained by treating IgG-type antibody molecule with a protease, pepsin.

The F (ab')₂ used in the present invention can be prepared by treating the antibody with a protease, pepsin. Alternatively, it can be prepared by linking Fab' described below via a thioether bond or a disulfide bond.

Fab' is an antibody fragment having a molecular weight of about 50,000 and exhibiting an antigen binding activity where a disulfide bond at a hinge region of the aforementioned F(ab')₂ is cleaved.

The Fab' used in the present invention can be prepared by treating F(ab')₂ with a reducing agent, dithiothreitol. Alternatively, DNA which codes for Fab' fragment of the antibody is inserted into expression vector for prokaryote or expression vector for eukaryote and the vector is introduced into the prokaryote or the eukaryote to express whereupon Fab' can be prepared.

scFv is an antibody fragment having an antigen binding activity and is an VH-P-VL or an VL-P-VH polypeptide where one VH and one VL are linked using an appropriate peptide linker (hereinafter, referred to as P).

The scFv used in the present invention can be prepared in such a manner that cDNA coding for VH and VL of the antibody is obtained, DNA coding for scFV is constructed, the DNA is inserted into expression vector for prokaryote or expression vector for eukaryote and the expression vector is introduced into the prokaryote or the eukaryote to express whereupon the scFc can be prepared.

Diabody is an antibody fragment where svFv is dimerized and is an antibody fragment having divalent antigen binding activity. The divalent antigen binding activity may be the same or one of them can be used as a different antigen binding activity. The diabody used in the present invention can be prepared in such a manner that cDNA coding for VH and VL of antibody is obtained, DNA coding for scFv is constructed so as to make the length of amino acid sequence of the linker to be not more than 8 residues, the DNA is inserted into expression vector for prokaryote or expression vector for eukaryote and the expression vector is introduced into the prokaryote or the eukaryote to express whereupon the diabody can be prepared.

The dsFv is an antibody fragment where a polypeptide in which each one amino acid residue in VH and VL is substituted with a cysteine residue is linked via a disulfide bond between the cysteine residues. The amino acid residue to be substituted with a cysteine residue can be selected based on a three-dimensional structure estimation of the antibody according to a method shown by Reiter, et al. (*Protein Engineering,* 7, 697-704, 1994). The dsFv used in the present invention can be prepared in such a manner that cDNA coding for VH and VL of an antibody is obtained, DNA coding for the dsFv is constructed, the DNA is inserted into expression vector for prokaryote or expression vector for eukaryote and the expression vector is introduced into the prokaryote or the eukaryote to express whereupon the dsFv can be prepared.

A CDR-containing peptide is constituted by comprising at least one region of CDRs of VH or VL. A peptide comprising plural CDRs can be linked either directly or via an appropriate peptide linker. A CDR-containing peptide used in the present invention can be prepared in such a manner that DNA coding for VH and VL of an antibody is obtained, the DNA is inserted into expression vector for prokaryote or expression vector for eukaryote and the expression vector is introduced into the prokaryote or the eukaryote to express whereupon the CDR-containing peptide is prepared. The CDR-containing peptide can also be prepared by a chemical synthetic method such as an Fmoc method (fluorenylmethyloxycarbonyl method) and a tBoc method (tert-butyloxycarbonyl method).

With regard to the antibody or the antibody fragment of the present invention, it is possible to evaluate a binding activity to IGF-I and IGF-II and an activity inhibiting the activity of IGF-I and IGF-II *in vitro* by ELISA (Antibodies: A Laboratory Manual, Cold Spring Harbor Laboratory, Chapter 14, 1988; Monoclonal Antibodies: Principles and Practice, Academic Press Limited, 1996) and by measuring an inhibitory activity of cell proliferation by IGF-I and IGF-II (*Cancer Research*, 48, 4083-4092, 1988), and the like.

### B. Preparation of active ingredient of the cancer metastasis inhibitor of the present invention

Hereunder, a process for producing the antibody or the antibody fragment being one of the antibodies used in the present invention which specifically binds to IGF-I and IGF-II and inhibits the activity of IGF-I and IGF-II and evaluation of activity thereof will be mentioned.

### 1. Preparation of a monoclonal antibody of non-human animal to IGF

### (1) Preparation of antigen

Expression vector comprising cDNA which codes for IGF is introduced and expressed in *Escherichia coli*, yeast, insect cell, animal cell, and the like to thereby obtain recombinant IGF protein and the resulting protein can be used as an antigen. Alternatively, a synthetic peptide having an IGF partial sequence can also be used as an antigen.

With regard to a partial peptide for antigen, a partial protein sequence of about 5 to 30 residues is selected. In order to obtain an antibody which recognizes the protein in a state of having a non-denatured authentic structure, it is necessary to select a partial sequence existing on the surface of protein in view of three-dimensional structure as an antigen peptide. The part existing on the surface of protein in view of three-dimensional structure can be presumed by predicting a highly hydrophilic partial sequence using commercially available software for analysis of protein sequence such as Genetyx Mac. Thus, that is because, in general, there are many cases where a lowly hydrophilic region is present in the inner part of the protein in view of three-dimensional structure and there are many cases where a highly hydrophilic region is present on the surface of protein. In addition, there are many cases where N-terminal and C-terminal of protein are present on the surface of protein. However, the partial peptide which is selected as such will not always be an antigen which establishes the desired antibody.

In order to cross-link to protein, cysteine is added to the terminal of a partial peptide. When an internal sequence of protein is selected as a partial peptide, N-terminal and C-terminal of the peptide are acetylated and amidated, respectively, if necessary. A partial peptide can be synthesized by a common liquid-phase or solid-phase peptide synthetic method, a method where they are appropriately combined or a modified method thereof (The Peptides, Analysis, Synthesis, Biology, Vol. 1, 1979; Vol. 2, 1980; Vol. 3, 1981, Academic Press; Fundamentals and Experiments for Peptide Synthesis, Maruzen, 1985; Development of Drugs, Second Series, Vol. 14, Peptide Synthesis, Hirokawa Shoten, 1991; *Intemational Journal of Protein* & *Protein Research*, 35, 161-214, 1990). It is also possible to use an automated peptide synthesizer. Synthesis of peptide using a peptide synthesizer can be carried out on a commercially available peptide synthesizer such as a peptide synthesizer manufactured by Shimadzu, a peptide synthesizer manufactured by Applied Biosystems, Inc. (hereinafter, referred to as ABI) and a peptide synthesizer manufactured by Advanced ChemTech Inc. (hereinafter, referred to as ACT) using Nα-Fmoc-amino acid or Nα-Boc-amino acid where side chain is appropriately protected according to synthetic program for each of them.

Protected amino acids used as raw material and carrier resins are available from ABI, Shimadzu, Kokusan Kagaku, Nova Biochem, Watanabe Kagaku, ACT, Peptide Laboratory, etc. Protected amino acids, protected organic acids and protected organic amines used as starting materials may also be synthesized by already-reported synthetic methods or modified methods thereof (The Peptides, Analysis, Synthesis, Biology, Vol. 1, 1979; Vol. 2, 1980; Vol. 3, 1981, Academic Press; Fundamentals and Experiments for Peptide Synthesis, Maruzen, 1985; Development of Drugs, Second Series, Vol. 14, Peptide Synthesis, Hirokawa Shoten, 1991; *International Journal* of *Protein & Protein Reserch,* 35, 161-214, 1990).

### (2) Immunization of animal and preparation of antibody-producing cell

With regard to the animal used for immunization, any animals may be used so long as it can prepare hybridoma such as mouse, rat, hamster and rabbit. Hereunder, examples using mouse and rat will be illustrated.

3 to 20-weeks old mice or rats were immunized with the antigen which was prepared in the aforementioned 1(1) and antibody-producing cells were collected from spleen, lymph node and peripheral blood of the animal. Immunization is carried out by administrating antigen to the animal for several times together with an appropriate adjuvant either subcutaneously, intravenously or intraperitoneally. Examples of the adjuvant are complete Freund's adjuvant or aluminum hydroxide gel, pertussis vaccine and the like. A conjugate is prepared with carrier protein such as bovine serum albumin (hereinafter, referred to as BSA) or keyhole limpet hemocyanin (hereinafter, referred to as KLH) and the resulting conjugate can be used as an immunogen. After 3 to 7 days from administrating each antigen, blood is collected from venous plexus of fundus of the eye or tail vein of the immunized animal, its reactivity to hIGF used as an antigen is confirmed by means of ELISA or the like and the mouse or rat where its serum shows a sufficient antibody value is used as a source for antibody-producing cell. On the 3 to 7 days from the final administrating the antigen, such as spleen, etc. are excised from the immunized mouse or rat according to a known method (Antibodies - A Laboratory Manual, Cold Spring Harbor Laboratory, 1988) and antibody-producing cells and myeloma cells are fused.

### (3) Preparation of myeloma cell

With regard to the myeloma cell, any myeloma cell may be used so long as it can proliferate *in vitro* such as 8-azaguanine-resistant myeloma cell line P3-X63Ag8-U1 (P3-U1) (*European Journal of Immunology*, 6, 511-519, 1976), SP2/0-Ag14 (SP-2) (*Nature,* 276, 269-270, 1978), P3-X63-Ag8653 (653) (*Journal of Immunology*, 123, 1548-1550, 1979), P3-X63-Ag8 (X63) (*Nature*, 256, 495-497, 1975) or the like. The culturing and sub-culturing of those cell lines can be carried out in accordance with a known method (Antibodies - A Laboratory Manual, Cold Spring Harbor Laboratory, 1988) to thereby secure and not less than 2 × 10⁷ cells until the stage of cell fusion.

### (4) Cell fusion

The antibody-producing cell and myeloma cell prepared hereinabove are washed and a cell-aggregating medium such as polyethylene glycol-1000 (hereinafter, referred to as PEG-1000) is added thereto whereupon cells are fused and suspended in a medium. For washing the cells, modified Eagle' s medium (hereinafter, referred to as MEM), phosphate buffered saline (hereinafter, referred to as PBS) or the like is used. With regard to a medium in which the fused cells are suspended, an HAT medium {a medium where 0.1 mM hypoxanthine, 15 µM thymidine and 0.4 µM aminopterin are added to a common medium [a medium where 1.5 mM glutamine, 50 µM 2-mercaptoethanol, 10 µg/mL gentamicin and 10% fetal bovine serum (hereinafter, referred to as FBS) are added to an RPMI-1640 medium]} is used so that the desired fused cell is selectively obtained.

After the culturing, a part of the culture supernatant liquid is taken out and a sample which reacts with antigen protein and does not react with non-antigen protein is selected by ELISA. After that, a limiting diluting method is carried out to make it into a single cell and a sample which showed a stable and high antibody titer by ELISA is selected as a monoclonal antibody-producing hybridoma.

### (5) Selection of hybridoma

Selection of hybridoma which produces an anti-hIGF monoclonal antibody is carried out by ELISA which will be mentioned later in accordance with a known method (Antibodies - A Laboratory Manual, Cold Spring Harbor Laboratory, 1988). According to such a method, it is now possible to measure a binding activity of antibody contained in a culture supernatant of transformant cell line which produces anti-hIGF chimeric antibody, anti-hIGF CDR-grafted antibody or antibody fragment thereof which will be mentioned later or all pure antibodies.

### ELISA

Antigen is fixed in a 96-well ELISA plate and reaction is carried out using a culture supernatant of such as hybridoma or purified antibody as the first antibody. After the reaction of the first antibody, the plate is washed and the second antibody is added. With regard to the second antibody, an antibody which can recognize the first antibody is labeled with biotin, enzyme, chemiluminescent substance, radioisotope or the like is used. To be more specific, when mouse is used in the preparation of the hybridoma, an antibody which can recognize the mouse antibody is used as the second antibody. After the reaction, the reaction corresponding to the labeled substance of the second antibody is carried out and selected as a hybridoma producing a monoclonal antibody which specifically reacts with the antigen.

A specific example of the hybridoma is a hybridoma KM 1468. The hybridoma KM 1468 has been deposited as FERM BP-7978 on March 26, 2002 under the stipulation of the Budapest Treaty at the International Patent Organism Depositary of the National Institute of Advanced Industrial Science and Technology (Central No. 6, 1-1, Higashi-1-chome, Tsukuba-Shi, Ibaragi-Ken, Postal Code; 305-8566).

### (6) Purification of monoclonal antibody

Anti-hIGF monoclonal antibody-producing hybridoma cell obtained in 1(4) are intraperitoneally injected in an amount of 5 × 10⁶ to 2 × 10⁷ cells/mouse to a mouse or nude mouse of 8 to 10-weeks old to which 0.5 mL of pristane (2,6,10,14-tetramethylpentadecane) is intraperitoneally administered followed by breeding for two weeks. With 10 to 21 days, the hybridoma becomes ascites tumor. The ascites is collected from the mouse or nude mouse, centrifuged, salting out with 40 to 50% saturated ammonium sulfate, subjected to a precipitation method with caprylic acid and IgG or IgM fraction is recovered by using DEAE-Sepharose column, protein A column, column of Cellulofine GSL 2000 (manufactured by Seikagaku Kogyo) or the like to prepare purified monoclonal antibody.

The subclass of the purified monoclonal antibody can be determined by using a mouse monoclonal antibody typing kit, a rat monoclonal antibody typing kit or the like. Concentration of protein can be calculated by a Lowry method or from the absorbance at 280 nm.

The subclass of the antibody means an isotype in the class and includes IgG1, IgG2a, IgG2b and IgG3 in the case of mouse and IgG1, IgG2, IgG3 and IgG4 in the case of human.

### (7) Activity evaluation of monoclonal antibody

### (7-1) Evaluation of binding activity to hIGF

Binding activity of the anti-hIGF monoclonal antibody which is in a culture supernatant or is purified can be measured by ELISA in the aforementioned 1(5), surface plasmon resonance (*Journal of Immunological Methods*, 145, 229-240, 1991), and the like. Reactivity with hIGF and antigen epitope can also be analyzed by a competitive ELISA using hIGF and partial peptides of hIGF. It can be presumed by a commonly conducted three-dimensional structural analytical method or combination of various immunological methods whether the antibody recognizes the three-dimensional structure of hIGF. Examples of the three-dimensional structural analytical method are X-ray crystallography and nuclear magnetic resonance method. An example of a combination of various immunological methods is a combination of ELISA to non-denatured antigen with ELISA to denatured antigen. In that case, the antibody shows reactivity only to non-denatured antigen, it is highly presumed that it recognizes the three-dimensional structure of the antigen. An example of ELISA to non-denatured antigen is ELISA where non-denatured antigen is allowed to react with antibody in a liquid phase. With regard to ELISA to denatured antigen, any method may be used so long as it is ELISA in which antibody is made toyreact under such a state that antigen does not have its authentic three-dimensional structure and its examples are ELISA to antigen which is directly fixed on a hydrophobic reaction plate and to partial peptide which is digested into an appropriate length.

The antibody of the present invention can be obtained by selecting an antibody having a binding activity to hIGF-II and a binding activity to hIGF-I by the measuring method for binding activity or by a competitive ELISA.

By examining the influence to cell line showing an hIGF-dependent proliferation, the inhibitory activity of the activity of hIGF *in vitro* can also be measured. Examples of cell line showing an hIGF-I- or hIGF-II-dependent proliferation are human breast cancer cell strain MCF7 (ATCC HTB-22), human colorectal cancer cell strain HT-29 (ATCC HTB-38), human prostate cancer cell strain MDA PCa 26 (ATCC CRL-2422) and the like.

Further, by establishing an hIGF-dependent cell proliferation measuring system using animal such as mice and examining influence on the measuring system, activity which inhibits the activity of hIGF *in vivo* can be measured. (7-2) Evaluation of inhibitory activity to metastasis of cancer

An example of an evaluating method for inhibitory activity to metastasis of cancer is a method using a metastasis evaluation system of human prostate cancer to human bone by Yonou, et al. (*Cancer Research*, 61, 2177-2182, 2001).

### 2. Preparation of polyclonal antibody of non-human animal to IGF

Polyclonal antibody can be prepared from serum of an animal where its serum shows a sufficient antibody titer among the animal to which immune is applied by the above method mentioned in 1.(2).

Thus, the serum fractionated by a centrifugation from the blood recovered from the animal or the immunoglobulin fraction is purified from the serum by a conventional method whereupon the polyclonal antibody can be prepared. With regard to activity of the polyclonal antibody, a binding activity to antigen can be evaluated by the above method mentioned in 1.(7).

### 3. Preparation of humanized antibody

### (1) Construction of vector for expression of humanized antibody

With regard to a vector for expression of humanized antibody, any vector for expression of humanized antibody may be used so long as it is vector for expression in animal cell into which gene coding for CH and/or CL of human antibody is inserted. Vector for expression of humanized antibody can be constructed by cloning the genes coding for CH and CL of human antibody, respectively, into vector for expression in animal cell.

C region of human antibody may be CH and CL of any human antibody and its examples are C region of IgG1 subclass of H chain of human antibody (hereinafter, referred to as hCγ1) and C region of κ class of L chain of human antibody (hereinafter, referred to as hCκ). With regard to the genes coding for CH and CL of human antibody, a chromosome DNA comprising exon and intron may be used and also, cDNA may be used.

With regard to vector for expression in animal cell, any vector may be used so long as it can be inserted and expressed the gene coding for the C region of human antibody. Its examples are pAGE107 (*Cytotechnology*, 3, 133-140, 1990), pAGE103 (*Journal of Biochemistry*, 101, 1307-1310, 1987), pHSG274 (*Gene*, 27, 223-232, 1984), pKCR (*Proceedings of the National Academy of Sciences of the United State of America*, 78, 1527-1531, 1981) and pSG1βd2-4 (*Cytotechnology*, 4, 173-180, 1990). Examples of promoter and enhancer used for the expression vector for animal cells are SV40 initial promoter and enhancer (*Journal of Biochemistry,* 101, 1307-1310, 1987), Moloney mouse leukemia virus *LTR* promoter and enhancer (*Biochemical & Biophysical Research Communications,* 149, 960-968, 1987) and immunoglobulin H chain promoter (*Cell,* 41, 479-487, 1985) and enhancer (*Cell*, 33, 717-728, 1983).

With regard to the vector for expression of human antibody, either of a type where antibody H chain and L chain are on different vectors and where they are on the same vector (hereinafter, referred to as a tandem type) may be used but, in view of easiness of construction of humanized antibody expression vector, easiness of introduction into animal cells and well-balanced expressed amount of antibody H chain and L chain in animal cells, a tandem type of vector for expression of humanized antibody is preferred (*Journal of Immunological Methods*, 167, 271-278, 1994). Examples of a tandem type of vector for expression of humanized antibody are pKANTEX93 (WO 97/10354) and pEE18 (*Hybridoma*, 17, 559-567, 1998).

The constructed vector for expression of humanized antibody can be used for expression of human chimeric antibody and human CDR-grafted antibody in animal cell.

### (2) Obtaining of cDNA coding for V region of antibody of non-human animal and analysis of amino acid sequence thereof

cDNA which codes for antibody of non-human animal such as VH and VL of mouse antibody is obtained as follows.

mRNA is extracted from hybridoma which produces mouse antibody, etc. and cDNA is synthesized. The synthesized cDNA is cloned into vector such as plasmid or phage to prepare a cDNA library. Using C region or V region of the mouse antibody as a probe, each of recombinant phage or recombinant plasmid having cDNA coding for VH or recombinant phage or recombinant plasmid having cDNA coding for VL is isolated from the library. Full length of nucleotide sequences of VH and VL of the desired mouse antibody on the recombinant phage or recombinant plasmid are determined and the full length of the amino acid sequences of VH and VL are deduced from the nucleotide sequences.

With regard to non-human animal, any animal such as mouse, rat, hamster and rabbit may be used so long as it can prepare a hybridoma.

An example of a method for preparing the total RNA from hybridoma is a guanidine thiocyanate-cesium trifluoroacetate method *(Methods in Enzmology,* 154, 3-28, 1987) and an example of a method for preparing mRNA from the total RNA is an oligo (dT) immobilized cellulose column method (Molecular Cloning: A Laboratory Manual, Cold Spring Harbor Lab. Press, New York, 1989). Examples of a kit for the preparing mRNA from hybridoma are Fast Track mRNA Isolation Kit (manufactured by Invitrogen), Quick Prep mRNA Purification Kit (manufactured by Pharmacia), and the like.

Examples of a method for the synthesizing of cDNA and for preparing cDNA library are a conventional method (Molecular Cloning: A Laboratory Manual, Cold Spring Harbor Lab. Press, New York, 1989; Current Protocols in Molecular Biology, Supplement 1-34) and a method using a commercially available kit such as Super Script™ Plasmid System for cDNA Synthesis and Plasmid Cloning (manufacture by Gibco BRL) and ZAP-cDNA Synthesis Kit (manufactured by Stratagene).

With regard to vector into which cDNA synthesized using mRNA extracted from the hybridoma as a template is inserted while preparing the cDNA library, any vector may be used so long as the cDNA can be inserted therein. For example, phage or plasmid vector such as ZAP Express (*Strategies*, 5, 58-61, 1992), pBluescript II SK(+) (*Nucleic Acid Research*, 17, 9494, 1989), λ ZAP II (manufactured by Stratagene), λ gt 10 and λ gt 11 (DNA Cloning: A Practical Approach, I, 49, 1985), Lambda BlueMid (manufactured by Clontech), λ ExCell, pT7T3 18U (manufactured by Pharmacia), pcD2 (*Molecular* & *Cellular Biology*, 3, 280-289, 1983) and pUC 18 (*Gene*, 33, 103-119, 1985) may be used.

With regard to *Escherichia coli* into which a cDNA library constructed by phage or plasmid vector is introduced, any *Escherichia coli* may be used so long as it the cDNA library can be inserted, expressed and maintained. Its examples are XL1-Blue MRF' (*Journal of Biotechnology*, 23, 271-289, 1992), C600 (*Genetics*, 59, 177-190, 1968), Y1088 and Y1090 (*Science*, 222, 778-782, 1983), NM 522 (*Journal of Molecular Biology*, 166, 1-19, 1983), K 802 (*Journal of Molecular Biology*, 16, 118-133, 1966), JM 105 (*Gene*, 38, 275-276, 1985) and the like.

With regard to a method for selecting cDNA clones coding for VH and VL of antibody of non-human animal from cDNA library, it can be selected by a colony hybridization method or a plaque hybridization method (Molecular Cloning: A Laboratory Manual, Cold Spring Harbor Lab. Press, New York, 1989) using radioisotope or fluorescence-labeled probe. In addition, cDNAs coding for VH and VL can be prepared by a polymerase chain reaction (hereinafter, referred to as PCR method; Molecular Cloning: A Laboratory Manual, Cold Spring Harbor Lab. Press, New York, 1989; Current Protocols in Molecular Biology, Supplement 1-34) by preparing primer and cDNA synthesized from mRNA or cDNA library as a template.

cDNA selected by the above-mentioned method is cleaved by an appropriate restriction enzyme or the like, cloned to a plasmid vector such as pBluescript SK(-) (manufactured by Stratagene), subjected to a method conventionally used for analysis of nucleotide sequence such as a dideoxy method (*Prooeedings of the National Academy of Sciences of the United States of America*, 74, 5463-5467, 1977) and analyzed by an automatic sequencer (ABI PRISM 377 (manufactured by ABI)) or the like whereupon a nucleotide sequence of the cDNA can be determined.

The full length of amino acid sequences of VH and VL are deduced from the determined nucleotide sequences and compared with the full length of amino acid sequences of VH and VL of known antibody (Sequences of Proteins of Immunological Interest, U. S. Dept. Health and Human Services, 1991) whereupon it can be confirmed whether the obtained cDNA codes for the full length of amino acid sequences of VH or VL of the antibody containing a signal sequence for secretion. With regard to the full length of amino acid sequences of VH or VL of the antibody containing a signal sequence for secretion, length and N-terminal amino acid sequence of the signal sequence can be deduced by comparing with the full length of amino acid sequences of VH and VL of the known antibody (Sequences of Proteins of Immunological Interest, U. S. Dept. Health and Human Services, 1991) and, further, subclass to which they belong can be determined. Also an amino acid sequence of each CDR of VH and VL can be found by comparing with the amino acid sequences of VH and VL of the known antibody (Sequences of Proteins of Immunological Interest, U. S. Dept. Health and Human Services, 1991).

A homology search of sequences such as a BLAST method (*Journal of Molecular Biology*, 215, 403-410, 1990) to any database such as SWISS-PROT or PIR-Protein can be conducted using the full length of amino acid sequences of VH and VL to examine novelty of the sequence.

### (3) Construction of human chimeric antibody expression vector

cDNAs coding for VH and VL of antibody of non-human animal are cloned to the upstream of genes coding for CH and CL of human antibody of vector for expression of humanized antibody mentioned in the above 3(1) to thereby construct human chimeric antibody expression vector. For example, each cDNA coding for VH and VL of antibody of non-human animal is ligated to synthetic DNA comprising a nucleotide sequence of 3' -terminal of VH and VL of antibody of non-human animal and a nucleotide sequence of 5'-terminal of CH and CL of human antibody and having recognition sequence of an appropriate restriction enzyme at both ends, and cloned so that each of them is expressed in an appropriate form on the upstream of gene coding for CH and CL of human antibody of the vector for expression of humanized antibody mentioned in the above 3(1) to construct human chimeric antibody expression vector. In addition, cDNA coding for VH and VL is amplified by a PCR method using a primer having a recognition sequence of an appropriate restriction enzyme at 5'-terminal using a plasmid containing cDNA coding for VH and VL of antibody of non-human animal and each of them is cloned so that it is expressed in an appropriate form at the upstream of gene coding for CH and CL of human antibody of the vector for expression of humanized antibody mentioned in the above 3(1) to construct human chimeric antibody expression vector.

### (4) Construction of cDNA coding for V region of human CDR-grafted antibody

cDNAs coding for VH and VL of human CDR-grafted antibody can be constructed as follows. Firstly, amino acid sequence of FRs in VH and VL of human antibody to which desired amino acid sequences of CDRs in VH and VL of non-human animal is selected. With regard to the amino acid sequence of FRs in VH and VL of human antibody, any amino acid sequence of FRs in VH and VL of human antibody may be used so long as it is derived from human antibody. Examples thereof are amino acid sequences of FRs in VH and VL of human antibody registered in database such as Protein Data Bank and a consensus amino acid sequence of each subgroup of FRs in VH and VL of human antibody (Sequences of Proteins of Immunological Interest, U. S. Dept. Health and Human Services, 1991). In order to prepare a human CDR-grafted antibody having a sufficient activity, an amino acid sequence having a homology of as high as possible (60% or more) to the amino acid sequence of FRs in VH and VL of antibody of desired non-human animal among the above is preferably selected. After that, the amino acid sequence of CDRs in VH and VL of desired non-human animal antibody is grafted to the selected amino acid sequence of FRs in VH and VL of the human antibody to design the amino acid sequences of VH and VL of the human CDR-grafted antibody. The designed amino acid sequences are converted to nucleotide sequences by considering the frequency of codon usage (Sequences of Proteins of Immunological Interest, U. S. Dept. Health and Human Services, 1991) found in the nucleotide sequence of gene of antibody whereupon nucleotide sequences coding for amino acid sequences of VH and VL of the human CDR-grafted antibody are designed. Based on the designed nucleotide sequences, several synthetic DNAs having a length of about 100 bases are synthesized and a PCR method is carried out by using them. In this case, it is preferred to design six synthetic DNAs for both VH and VL in view of reaction efficiency in the PCR and length of synthesizable DNA.

Further, by introducing a recognition sequence of an appropriate restriction enzyme into 5'-terminal of synthetic DNAs located at both ends, cloning to a vector for expression of humanized antibody constructed in the above 3(1) can be carried out. After the PCR, the amplified product is cloned to a plasmid such as pBluescript SK(-) (manufactured by Stratagene) and a nucleotide sequence is determined by the method mentioned in the above 3(2) whereupon a plasmid having nucleotide sequences coding for the amino acid sequences of VH and VL of the desired human CDR-grafted antibody is obtained.

### (5) Modification of amino acid sequence of V region of human CDR-garafted antibody

It has been known that, when a human CDR-garafted antibody id produced by simply grafting the target CDRs in VH and VL of an antibody of the non-human animal into FRs in VH and VL of human antibody, antigen-binding antibody of human CDR-grafted antibody lowers as compared with the original antibody of the non-human animal (*Bio*/*Technology*, 9, 266-271, 1991). With regard to the cause thereof, it is considered that, in the original VH and VL of antibody of the non-human animal, not only CDRs but also various amino acid residues of FRs participate in antigen-binding activity either directly or indirectly and that, as a result of grafting of CDRs, such amino acid residues change to amino acid residues being different from FRs in VH and VL of the human antibody. In order to solve the problem, it has been conducted in a human CDR-grafted antibody that, among the amino acid sequence of FRs in VH and VL of human antibody, an amino acid residue which directly relates to binding to the antigen, or amino acid residue which indirectly relates to binding to an antigen by interacting with an amino acid residue in CDRs or by maintaining the three-dimensional structure of an antibody, is identified and that the amino acid residues is modified to amino acid residues found in the original antibody of non-human animal to thereby increase the lowered antigen-binding activity (*Bio*/*Technology*, 9, 266-271, 1991). In the preparation of human CDR-grafted antibody, how to efficiently identify the amino acid residues of relating to the antigen binding activity in FR is most important, so that the three dimensional structure of an antibody is constructed and analyzed by X-ray crystallography (*Journal of MolecularBiology*, 112, 535-542, 1977), a computer-modeling (*Protein Engineering*, 7, 1501-1507, 1994), or the like. Information for three-dimensional structure of the antibody as such has given much advantageous information to the preparation of human CDR-grafted antibody but, on the other hand, no method for the preparing of human CDR-grafted antibody which is applicable to any antibodies has not been established yet and, at present, various trials and errors are necessary such as that several kinds of modified products are prepared for each antibody and that correlation to each antigen binding activity is examined.

Modification of amino acid residue of FRs in VH and VL of human antibody can be achieved by conducting a PCR method mentioned in the above 3(4) using a synthetic DNA for the modification. With regard to the amplified product after the PCR, its nucleotide sequence is determined by the method mentioned in the above 3(2) whereby it is confirmed that the desired modification has been done.

### (6) Construction of the human CDR-grafted antibody expression vector

cDNAs coding for VH and VL of the human CDR-grafted antibody constructed in the above 3(4) and (5) are cloned to the upstream of genes coding for CH and CL of the human antibody in the vector for expression of the humanized antibody mentioned in the above 3(1) to thereby construct a human CDR-grafted antibody expression vector.

For example, in the synthetic DNA used for the construction of VH and VL of the human CDR-grafted antibody in the above 3(4) and (5), recognition sequences of an appropriate restriction enzyme are introduced into 5' -terminal of the synthetic DNAs located at both ends whereby they can be cloned to the upstream of genes coding for CH and CL of human antibody in the vector for expression of humanized antibody mentioned in the above 3(1) in such a manner that they are expressed in an appropriate form.

### (7) A transient expression of humanized antibody

In order to efficiently evaluate the antigen-binding activity of the various humanized antibodies prepared, a transient expression of humanized antibody can be conducted using the humanized antibody expression vector mentioned in the above 3(3) and (6) or the modified expression vector thereof. With regard to a host cell into which the expression vector is introduced, any cell may be used so long as it is a host cell which can express the humanized antibody, but COS-7 cell (ATCC CRL-1651) has been commonly used in view of its high expressing amount (Methods in Nucleic Acids Research, CRC Press, 283, 1991). The methods for the introducing the expression vector into COS-7 cells are DEAE-dextran method (Methods in Nucleic Acids Research, CRC Press, 283, 1991), a lipofection method (*Proceedings of the National Academy of Sciences of the United States of America*, 84, 7413-7417, 1987), and the like.

After introducing the expression vector, the amount of humanized antibody expressed in the culture supernatant and antigen-binding activity can be measured by, for example, ELISA (Antibodies: A Laboratory Manual, Cold Spring Harbor Laboratory, Chapter 14, 1988; Monoclonal Antibodies: Principles and Practice, Academic Press Limited, 1996).

### (8) Stable expression of humanized antibody

A transformant cell which stably expresses humanized antibody can be obtained by introducing the humanized antibody expression vector mentioned in the above 3(3) and (6) into appropriate host cell. The methods for the introducing expression vector into host cell are an electroporation method (*Cytotechnology*, 3, 133-140, 1990), and the like. With regard to the host cell into which humanized antibody expression vector is introduced, any cell may be used so long as it is a host cell which can express the humanized antibody. Examples thereof are mouse SP2/0-Ag14 cell (ATCC CRL-1581), mouse P3X63-Ag8.653 cell (ATCC CRL-1580), dihydrofolate reductase gene (hereinafter, referred to as *dhfr*)-deficient CHO cell (*Proceedings of the National Academy of Scienoes of the United States of America*, 77, 4216-4220, 1980) and rat YB2/3HL.P2.G11.16Ag.20 cell (ATCC CRL-1662; hereinafter, referred to as YB2/0 cell).

A transformant in which humanized antibody is stably expressed after introducing expression vector can be selected by culturing in a medium for animal cell culture containing an agent such as G418 sulfate (hereinafter, referred to as G418) according to a process disclosed in Japanese Published Unexamined Patent Application NO. 257891/90. With regard to a medium for culturing animal cell, RPMI 1640 medium (manufactured by Nissui Seiyaku), GIT medium (manufactured by Nippon Seiyaku), EX-CELL 302 medium (manufactured by JRH), IMDM (manufactured by Gibco BRL), Hybridoma-SFM (manufactured by Gibco BRL), a medium obtained by adding various additives such as FBS thereto, and the like may be used. When the resulting transformant cell is cultured in a medium, humanized antibody can be expressed and accumulated in the culture supernatant. The amount of the humanized antibody expressed in the culture supernatant and antigen-binding activity can be measured by ELISA. Further, in the transformant cell, the amount of the humanized antibody expressed can be increased by utilizing a *dhfr* system or the like according to a method disclosed in Japanese Published Unexamined Patent Application NO. 257891/90.

Humanized antibody can be purified from the culture supernatant of the transformant cell using a protein A column (Antibodies: A Laboratory Manual, Cold Spring Harbor Laboratory, Chapter 8, 1988; Monoclonal Antibodies: Principles and Practice, Academic Press Limited, 1996). Besides that, purifying methods which are usually used for purification of proteins can be used. For example, gel filtration, ion-exchange chromatography and ultrafiltration may be conducted in combination so as to purify. Molecular weight of H chain and L chain of the purified humanized antibody or of the whole antibody molecular can be determined by a polyacrylamide gel electrophoresis (hereinafter, referred to as PAGE; *Nature*, 227, 680-685, 1970), a western blotting method (Antibodies: A Laboratory Manual, Cold Spring Harbor Laboratory, Chapter 12, 1988; Monoclonal Antibodies: Principles and Practice, Academic Press Limited, 1996), and the like.

### (9) Evaluation of activity of humanized antibody

Evaluation of activity of humanized antibody can be carried out in the same manner as in the above 1(7).

### 4. Preparation of antibody fragment

Antibody fragment can be prepared from the anti-hIGF antibody mentioned in the above 1 and 2 by genetic engineering techniques or protein chemical techniques.

Examples of the genetic engineering techniques are a method where gene coding for desired antibody fragment is constructed and expression and purification are conducted using a suitable host such as animal cells, plant cells, insect cells, *Escherichia coli*, or the like.

Examples of the protein chemical techniques are a method of site-specific cleavage, purification using a protease such as pepsin and papain, and the like.

A process for producing of Fab, F(ab')₂, Fab', scFv, diabody, dsFv or CDR-containing peptide as a antibody fragment will be specifically illustrated as follows.

### (1) Preparation of Fab

Fab can be prepared by treating IgG with protease, papain by using protein chemical techniques. After the treatment with papain, it is possible to recover as a uniform Fab by passing through a protein A column to separate from IgG molecule and Fc fragment provided that the original antibody is an IgG subclass having a binding property to protein A (Monoclonal Antibodies: Principles and Practice, third edition, 1995). In the case of an antibody of an IgG subclass having no binding property to protein A, Fab can be recovered by an ion-exchange chromatography at a fraction eluted by a low salt concentration (Monoclonal Antibodies: Principles and Practice, third edition, 1995). Fab can also be prepared by genetic engineering techniques using *E. coli* in many cases or using insect cells, animal cells, and the like. For example, DNA coding for V region of the antibody mentioned in the above 3(2), 3(4) and 3(5) is cloned to a vector for expression of Fab whereupon Fab expression vector can be prepared. With regard to vector for expression of Fab, any vector may be used so long as DNA for Fab can be inserted and expressed. An example thereof is pIT 106 (*Science*, 240, 1041-1043, 1988). Fab expression vector is introduced into an appropriate *E. coli* whereby Fab can be formed and accumulated in an inclusion body or a periplasmic space. From the inclusion body, active Fab can be obtained by a refolding method generally used for proteins and, when expressed in periplasmic space, active Fab leaks out in a culture supernatant. After the refolding or from the culture supernatant, a uniform Fab can be purified using a column to which antigen is bound (Antibody Engineering, A Practical Guide, W. H. Freeman and Company, 1992).

### (2) Preparation of F(ab')2

F(ab')₂ can be prepared by treating of IgG with protease, pepsin by using protein chemical techniques. After the treatment with pepsin, it can be recovered as a uniform F(ab')₂ by the same purifying operation as in the case of Fab (Monoclonal Antibodies: Principles and Practice, third edition, Academic Press, 1995). It can also be prepared by a method where Fab' mentioned in the following 4(3) is treated with a maleimide such as o-PDM or bismaleimide to form a thioether bond or by a method where it is treated with DTNB [5,5'-dithiobis(2-nitrobenzoic acid)] to form an S-S bond (Antibody Engineering, A Practical Approach, IRL Press, 1996).

### (3) Preparation of Fab'

Fab' can be prepared by treating F(ab')₂ mentioned in the above 4(2) with a reducing agent such as dithiothreitol. Fab' can be prepared by genetic engineering techniques using *E*. *coli* in many cases or using insect cells, animal cells, and the like. For example, DNA coding for V region of the antibody mentioned in the above 3(2), 3(4) and 3(5) is cloned to a vector for expression of Fab' whereupon Fab' expression vector is able to be prepared. With regard to a vector for expression of Fab' , any vector may be used so long as DNA for Fab' can be inserted and expressed. An example thereof is pAK 19 (*Bio*/*Technology*, 10, 163-167, 1992). The Fab' expression vector is introduced into an appropriate *E. coli* to form and accumulate Fab' in an inclusion body or periplasmic space. From the inclusion body, active Fab' can be obtained by a refolding method which is usually used in proteins and, when the Fab' is expressed in periplasmic space, it can be recovered extracellulary by disrupting the cell with treating such as partial digestion by lysozyme, osmotic shock and sonication. After the refolding or from the disrupted cell solution, a uniform Fab' can be purified using a protein G column or the like (Antibody Engineering, A Practical Approach, IRL Press, 1996).

### (4) Preparation of scFv

scFv can be prepared using phage or *E*. *coli* or using insect cells or animal cells by genetic engineering techniques. For example, DNA coding for V region of the antibody mentioned in the above 3(2), 3(4) and 3(5) is cloned to a vector for expression of scFv whereupon an scFv expression vector is able to be prepared. With regard to the vector for expression of scFv, any vector may be used so long as the DNA of scFv can be inserted and expressed. Examples thereof are pCANTAB5E (manufactured by Pharmacia), pHFA (*Human Antibodies* & *Hybridomas*, 5, 48-56, 1994), and the like. When scFv expression vector is introduced into an appropriate *E. coli* and a helper phage is infected, to thereby obtain a phage which expresses scFv on the phage surface in a fused form with the surface protein of the phage. Also, scFv can be formed and accumulated in periplasmic space or an inclusion body of *E*. *coli* into which scFv expression vector is introduced. From the inclusion body, active scFv can be obtained by a refolding method generally used for proteins and, when scFv is expressed in periplasmic space, it can be recovered extracellulary by disrupting the cell with treating such as partial digestion by lysozyme, osmotic shock and sonication. After the refolding or from the disrupted cell solution, a uniform scFv can be purified using a cation-exchange chromatography or the like (Antibody Engineering, A Practical Approach, IRL Press, 1996).

### (5) Preparation of diabody

Diabody can be prepared using *E. coli* in many cases or using insect cells, animal cells, and the like by genetic engineering techniques. For example, DNAs in which VH and VL of the antibody mentioned in the above 3(2), 3(4) and 3(5) are linked by a linker coding 8 amino acid residues or less is prepared and cloned into a vector for expression of diabody whereupon a diabody expression vector can be prepared. With regard to a vector for expression of diabody, any vector may be used so long as the DNA of diabody can be inserted and expressed. Examples thereof are pCANTAB 5E (manufactured by Pharmacia) and pHFA (*Human Antibodies Hybridomas*, 5, 48, 1994). Diabody can be formed and accumulated in periplasmic space or inclusion body of *E. coli* into which a diabody expression vector is introduced. From the inclusion body, active diabody can be obtained by a refolding method generally used for proteins and, when the diabody is expressed in periplasmic space, it can be recovered extracellulary by disrupting the cell with treating such as partial digestion by lysozyme, osmotic shock and sonication. After the refolding or from the disrupted cell solution, a uniform scFv can be purified using a cation-exchange chromatography or the like (Antibody Engineering, A Practical Approach, IRL Press, 1996).

### (6) Preparation of dsFv

dsFv can be prepared using *E*. *coli* in many cases or using insect cells, animal cells, and the like by genetic engineering techniques. Firstly, mutation is introduced into an appropriate position of DNA coding for VH and VL of the antibody mentioned in the above 3(2), 3(4) and 3(5) to prepare DNAs in which an encoded amino acid residue is replaced with cysteine. Each DNA prepared as such is cloned to a vector for expression of dsFv whereby an expression vector of VH and VL can be prepared. With regard to a vector for expression of dsFv, any vector may be used so long as the DNA for dsFv can be inserted and expressed. An example thereof is pULI 9 (*Protein Engineering*, 7, 697-704, 1994). The expression vector of VH and VL is introduced into an appropriate *E. coli* and dsFv is formed and accumulated in an inclusion body or periplasm. VH and VL are obtained from the inclusion body or periplasm, mixed and subjected to a refolding method generally used for proteins to thereby obtain active dsFv. After the refolding, it can be further purified by an ion-exchange chromatography, a gel filtration, and the like. (*Protein Engineering,* 7, 697-704, 1994).

### (7) Preparation of CDR-containing peptide

CDR-containing peptide can be prepared by a chemical synthesis method such as an Fmoc method or a tBoc method. Further, DNA coding for a CDR-containing peptide is prepared and the resulting DNA is cloned to an appropriate vector for expression whereby a CDR-containing peptide expression vector can be prepared. With regard to a vector for expression, any vector may be used so long as the DNA which codes for CDR-containing peptide can be inserted and expressed. Examples thereof are pLEX (manufactured by Invitrogen) and pAX4a+ (manufactured by Invitrogen). The expression vector is introduced into an appropriate *E*. *coli* and formed and accumulated in an inclusion body or periplasmic space. From the inclusion body or the periplasm, CDR-containing peptide is obtained and can be purified by an ion-exchange chromatography and a gel filtration (*Protein Engineering*, 7, 697-704, 1994).

### (8) Evaluation of activity of antibody fragments

Evaluation of activity of the purified antibody fragment can be carried out in the same manner as in the above-mentioned 1(7).

### C. Cancer metastasis Inhibitor of the present invention

A substance which inhibits the activity of IGF-I and IGF-II or, to be more specific, an antibody or an antibody fragment which specifically binds to IGF-I and IGF-II prepared as above and inhibits the activity of IGF-I and IGF-II has an inhibitory activity for metastasis of cancer and, therefore, it is useful as an cancer metastasis inhibitor. In addition, the antibody or the antibody fragment has an activity of suppressing the progress of a morbid state even to metastatic focus where, after the metastasis, proliferation starts at the metastatic focus and morbid state progresses and, therefore, it is also useful as a pharmaceutical composition for metastatic focus.

With regard to the disease which is an object of the inhibitor for metastasis of cancer of the present invention, there is no particular limitation so long as its metastasis from primary focus is statistically highly frequent and its examples are various kinds of malignant and benign tumor such as malignant melanoma, malignant lymphoma, gastral cancer, lung cancer, esophageal cancer, stomach cancer, colorectal cancer, rectum cancer, colic cancer, ureteral tumor, cystic cancer, cholangioma, biliary cancer, breast cancer, hepatic cancer, pancreatic cancer, testicular tumor, palatitis, cancer of the tongue, canker cancer, cancrum, pharyngeal cancer, laryngeal cancer, ovarian cancer, uterine cancer, prostate cancer, thyroid cancer, brain tumor, Kaposi's sarcoma, hemangioma, leukemia, erythrosis, neuroblastoma, retinoblastoma, myeloma, bladder tumor, sarcoma, osteosarcoma, myosarcoma and skin cancer.

The metastasis cancer inhibitor of the present invention is particularly effective for "hIGF-dependently proliferated cancer". "hIGF-dependently proliferated cancer" means cancer which is proliferated in the presence of hIGF and its degree of proliferation increases dependent upon the concentration of hIGF. Its examples are prostate cancer, colorectal cancer, breast cancer and osteosarcoma.

With regard to the metastatic focus to which the aforementioned various malignant and benign tumors are metastasized, any organ may be included therein so long as it is an organ where metastasis of cancer is seen in human organ. Examples of organ where metastasis is frequently seen among the above are liver, kidney, stomach, colon, pancreas, lacteal gland, prostate gland, bladder, lung, bronchus, brain, nerve, lymph node, adrenal gland, bone or the like.

Since the cancer metastasis inhibitor of the present invention is effective for metastasis of "hIGF-dependently proliferated cancer", it is particularly effective for metastasis of cancer to the organ where hIGF is present therein among the aforementioned organs and examples of such an organ where hIGF is present therein are liver, bone or the like.

Metastasis of cancer includes not only unsympatric metastasis which is mediated by blood vessel and lymph vessel but also sympatric metastasis which is not mediated by blood vessel or lymph vessel.

Examples of the specific metastasis in the present invention are bone metastasis of prostate cancer, liver metastasis of colorectal cancer, bone metastasis of breast cancer and bone metastasis of osteosarcoma such as multiple myeloma.

The cancer metastasis inhibitor of the present invention may comprise antibody or antibody fragment which is a substance (hereinafter, referred to as an inhibiting substance) inhibiting the activity of IGF-I and IGF-II solely as active ingredient but, usually, it is preferred to be mixed with one or more pharmaceutically acceptable carriers and is provided as a pharmaceutical composition which is manufactured by any method well known in the technical field of pharmaceutical compositions. Preferably, an aseptic solution where it is dissolved in an aqueous carrier such as water, and an aqueous solution of salt, glycine, glucose or human albumin are used. It is also possible to add a pharmaceutically acceptable additive such as buffer or isotonizing agent for making the preparation solution more similar to the physiological conditions and examples thereof are sodium acetate, sodium chloride, sodium lactate, potassium chloride and sodium citrate. It may also be preserved by freeze-drying and, in actual use, it may be used by dissolving in an appropriate solvent.

With regard to the administration route of the cancer metastasis inhibitor of the present invention, it is preferred to use the most effective route for the treatment. Examples thereof are oral administration and parenteral administration such as intraoral, tracheobronchial, intrarectal, subcutaneous, intramuscular, intraarticular and intravenous, and, among them, intravenous administration is preferred.

Examples of the preparation suitable for the oral administration are emulsion, syrup, capsule, tablet, diluted powder and granule. Liquid preparation such as emulsion and syrup can be prepared using water, saccharides such as sucrose, sorbitol and fructose, glycols such as polyethylene glycol and propylene glycol, oils such as sesame oil, olive oil and soybean oil, antiseptics such as p-hydroxybenzoate, flavors such as strawberry flavor and peppermint flavor and the like as additives. Capsule, tablet, diluted powder, granule, and the like can be prepared using excipients such as lactose, glucose, sucrose and mannitol, disintegrating agents such as starch and sodium alginate, lubricants such as magnesium stearate and talc, binders such as polyvinyl alcohol, hydroxypropyl cellulose and gelatin, surfactants such as fatty acid ester, plasticizers such as glycerol, as additives.

Examples of the preparation suitable for parenteral administration are injection, suppository and air spray. For example, injection is prepared using a carrier comprising salt solution, glucose solution or a mixture of both, or the like. Suppository is prepared using a carrier such as cacao butter, hydrogenated fat or carboxylic acid. Air spray is prepared using the inhibiting substance as such or using, for example, a carrier which does not stimulate the mouth and the airway mucous membrane of a person to be administered, and which disperses the inhibiting substance into fine particles and makes the absorption easy. Specific examples of the carrier are lactose and glycerol. Depending upon the property of the inhibiting substance and the carrier used, it is possible to prepare aerosol, dry powder, and the like. In addition, even in the parenteral preparation, components exemplified as additives in the oral preparation may be added.

Dose and the number of administration of the cancer metastasis inhibitor of the present invention vary depending upon desired therapeutic effect, administering method, period for the treatment, age, body weight, and the like and, usually, it is 10 µg/kg to 10 mg/kg per day for an adult.

A person to whom the cancer metastasis inhibitor of the present invention is administered is a patient suffering from cancer where primary focus and metastatic focus are clinically confirmed either simultaneously or solely for each of them and is unrelated to the fact whether any of surgical therapy, chemotherapy, radiotherapy and all other therapeutic methods for cancer are applied.

Even in the case of a patient suffering from cancer where only primary focus is clinically seen, there are some cases where there is metastasis which is difficult to clinically find in the focus other than the primary focus. The metastasis which is difficult to clinically find as such is a cause for regeneration of cancer after the treatment of the primary focus. Since the cancer metastasis inhibitor of the present invention is effective for metastatic focus of the cancer, it may be used as a preventive agent for regeneration after metastasis.

### Best Mode for Carrying Out the Invention

The present invention will now be illustrated in detail according to the following Examples although those Examples are never indeed to limit the present invention.

### (Example 1) Examination of inhibitory effect for prostate cancer cell line metastasis to bone by anti-hIGF antibody KM 1468

### (1) Influence of hIGF-I on proliferation of human prostate cancer cell line MDA PCa 2b cells

Prior to the examination of inhibition for cancer metastasis, it was examined whether proliferation of human prostate cancer cell line MDA PCa 2b cell (ATCC CRL-2422) is affected by hIGF-I.

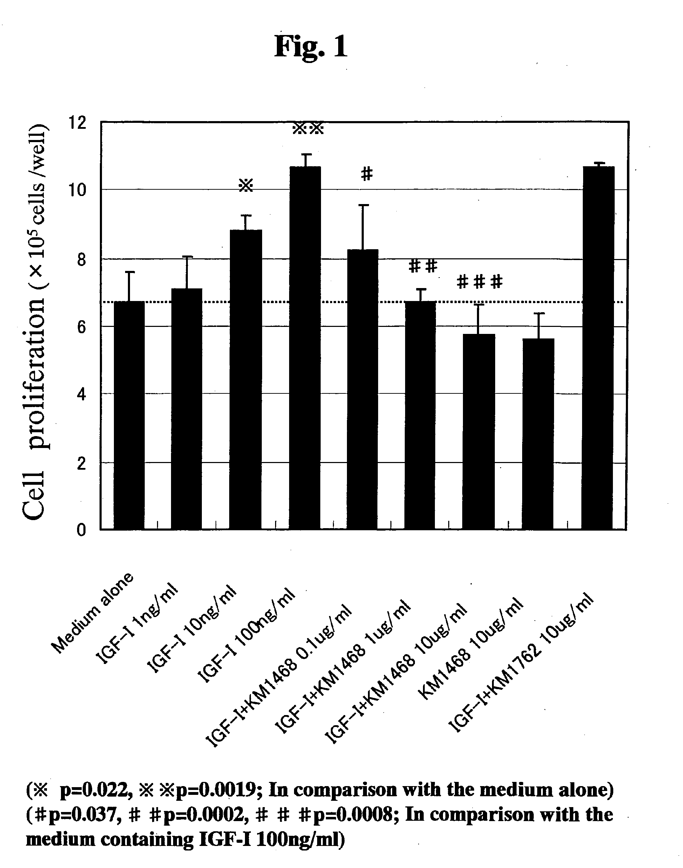

MDA PCa 2b cell (5 × 10⁵ cell/well) were inoculated to a 6-well plate and subjected to a stationary culture in an incubator with 5% CO₂ at 37°C using an F12K (+) medium {25 ng/mL cholera toxin (manufactured by List Biological Laboratories), 10 ng/mL of recombinant human epithelial growth factor (manufactured by Invitrogen), 100 pg/mL of hydrocortisone (manufactured by Sigma), 0.1 nM dihydrotestosterone (manufactured by Sigma) and 1/100 volume of ITS-X supplement (x 100) (manufactured by Gibco BRL) were added to F12K (manufactured by Sigma)} containing a 20% fetal bovine serum (manufactured by Sigma). After the incubation for 48 hours, a medium in each well was exchanged for a serum-free F12K medium (manufactured by Sigma) to which no or 1, 10 or 100 ng/mL of hIGF-I (manufactured by R & D) was added. After a stationary culturing for 48 hours in a 50% CO₂ incubator at 37°C since the exchange for a serum-free F12K medium, cells were recovered and numbers of living cell in each well were measured by staining with trypan blue. The result is shown in Fig. 1.

As shown in Fig. 1, it is now apparent that proliferation of MDA PCa 2b cell was promoted in a manner depending upon hIGF-I concentration.

### (2) Influence of anti-hIGF antibody KM 1468 on the hIGF-I-dependent cell proliferation

It was examined whether the hIGF-I-dependent cell proliferation was affected by anti-hIGF antibody KM 1468 (hereinafter, referred to as antibody KM 1468) using MDA PCa 2b cell which proliferate in an hIGF-I-dependent manner mentioned in the above (1). The antibody KM 1468 was prepared according to reference Example 1 which will be described later.

After the MDA PCa 2b cell were incubated for 48 hours in a 5% CO₂ incubator at 37°C the same as in Example 1(1), the medium was exchanged for a serum-free F12K medium to which 100 ng/mL of hIGF-I (manufactured by R & D) and 0, 0.1, 1 or 10 µg/mL (in terms of the final concentration) of the antibody KM 1468 were each added and a stationary culture was carried out in a 5% CO₂ incubator at 37°C. After 48 hours from for the exchange to the serum-free medium, cell were recovered and the number of living cell in each well were measured by staining with trypan blue. The result is shown in Fig. 1.

As shown in Fig. 1, the antibody KM 1468 inhibited the hIGF-I-dependent proliferation of the MDA PCa 2b cell. The proliferation inhibitory activity was dependent upon the concentration of the antibody KM 1468. An antibody KM 1762 (anti-avermectin antibody) which was a negative control did not inhibit the proliferation at all.

### (3) Examination of inhibitory effect of antibody KM 1468 for bone metastasis of prostate cancer cell line and therapeutic effect thereof on metastatic focus

Inhibitory effect of anti-hIGF antibody for bone metastasis and therapeutic effect for metastatic focus were examined using a human bone metastasis model of human prostate cancer cell line MDA PCa 2b cell.

Human bone tissue piece of about 1 cm³ prepared according to a method of Yonou, et al. (*Cancer Research*, 61, 2177-2182, 2001) was grafted subcutaneously to NOD/SCID mice of 6 to 8-weeks old (Nippon Claire). After 4 weeks from the grafting of the human bone tissue piece, human prostate cancer cell line MDA 2Ca 2b cell were suspended in a serum-free medium at the density of 4 × 10⁷ cell/mL and 100 µL of the cell suspension was injected into the grafted human bone tissue piece. In a early metastasis model, administration of the antibody was started simultaneously with the grafting of the human prostate cancer cell line MDA PCa 2b cell. On the other hand, in a late metastasis model, administration of the antibody was started after 4 weeks from the grafting of the human prostate cancer cell line MDA PCa 2b cell.

The mice in each model were classified into a mouse group to which the antibody KM 1468 was administered (hereinafter, referred to as a treated group) and a mouse group to which an antibody KM 1762 (anti-avermectin antibody) was administered as a negative control (hereinafter, referred to as a non-treated group) and administration of the antibody to the mice of each group intraperitoneally was carried out once a week for four weeks. Dose of the antibody KM 1468 per administration in the treated group was 0.01, 0.1 or 2 mg/kg in the early metastasis model while, in the late metastasis model, it was 0.1, 2 or 10 mg/kg. Dose of the antibody KM 1762 per administration in the non-treated group was 2 mg/kg in the early metastasis model while, in the late metastasis model, it was made 10 mg/kg. The Number of the mice tested at the same time were 9 to 10 in both treated and non-treated groups. In both models, the grafted bone tissues were excised after 4 weeks from the start of the administration and collected the whole blood at the same time.

The excised human bone tissue was made into slices and the area occupied by tumor cell in the bone tissue was analyzed using an image analyzer KS 300 system, version 2.00 (manufactured by Carl Zeiss). Further, serum fraction was fractionated from the collected blood and concentration of prostatic specific antigen (hereinafter, referred to as PSA) which is a serum tumor marker for prostate cancer was measured using a radioimmunoassay kit (manufactured by Hybritech).

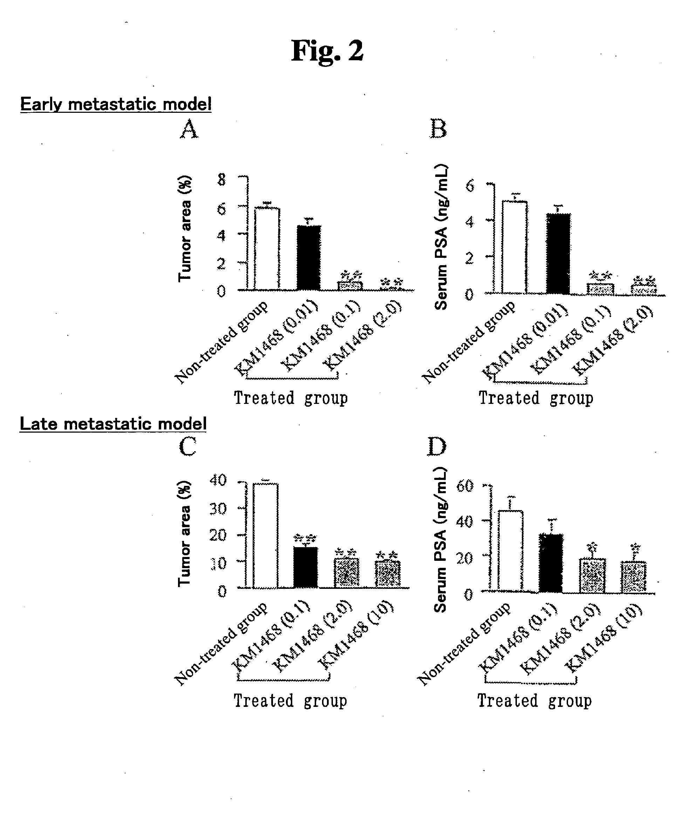

Ratio of the area of the metastasized tumor cell in the human bone tissue excised from the mice of the early metastasis model is shown in Fig. 2A while ratio of the area of the metastasized tumor cell in the human bone tissue excised from the mice of the late metastasis model is shown in Fig. 2C. In the non-treated group of the early metastasis model (Fig. 2A), the ratio of the tumor area in the human bone tissue was 6% while, in the treated group of the same model, the ratio of the tumor area decreased in a dose dependent manner of the amount of the administered antibody as compared with the non-treated group. In the maximum dose of 2 mg/kg, the ratio of the tumor area decreased to about 1/20 as compared with the non-treated group. Similarly, in the late metastasis model (Fig. 2C), the ratio of the tumor area in the bone tissue was 38% in the non-treated group while, in the treated group of the same model, the ratio of the tumor area decreased in a dose dependent manner of the amount of the administered antibody as compared with the non-treated group. In the maximum dose of 10 mg/kg, the ratio of the tumor area decreased to about 1/4 as compared with the non-treated group.

PSA concentration in serum of the mice of the early metastasis model is shown in Fig. 2B while PSA concentration in serum of the mice of the late metastasis model is shown in Fig. 2D. In the non-treated group of the early metastasis model (Fig. 2B), the PSA concentration in serum was about 5 ng/mL while, in the treated group of the same model, the PSA in serum decreased in a dose dependent manner of the amount of the administered antibody as compared with the non-treated group. In the maximum dose of 2 mg/kg, the serum PSA decreased to about 1/10 as compared with the non-treated group. Similarly, in the late metastasis model (Fig. 2D), the concentration of PSA in serum was about 45 ng/mL in the non-treated group while, in the treated group of the same model, the serum PSA decreased in a dose dependent manner of the amount of the administered antibody as compared with the non-treated group. In the maximum dose of 10 mg/kg, the serum PSA decreased to about 1/2 as compared with the non-treated group.

From the above results, it was shown that the antibody KM 1468 not only inhibited the adhesion of the human prostate cancer cell strain MDA PCa 2b cell to human bone tissue grafted into the body of mice but also had an activity of inhibiting the proliferation of cancer cell which were proliferated with a lapse of time after the adhesion. Accordingly, it was made clear that the antibody KM 1468 not only inhibits the bone metastasis of prostate cancer but also has an effective therapeutic effect for metastasitic focus.

### (Example 2) Examination of inhibitory effect for bone metastasis of multiple myeloma cell line by the antibody KM 1468

### (1) Influence on human multiple myeloma cell line RPMI 8226 cell by stimulation with hIGF-I

Prior to a test for inhibitory effect for bone metastasis, an influence by hIGF-I stimulation on RPMI 8226 cell (ATCC CCL-155) which is a human multiple myeloma cell line was examined by a western blotting (Antibodies: A Laboratory Manual, Cold Spring Harbor Laboratory, Chapter 12, 1988). At that time, an antibody which specifically reacts with IGF-IR, Akt (another name: protein kinase B) and mitogen-activated protein kinase (hereinafter, referred to as MAPK) each being phosphorylated was used to detect the phosphorylation of IGF-IR, Akt and MAPK.

After incubation of RPMI 8226 cell each being 6 × 10⁶ cells for 24 hours under a serum-free condition, hIGF-I (manufactured by R & D) was added at a concentration of 0, 1 or 10 ng/mL and stimulation was applied for 30 minutes. Cell after the stimulation were washed with a cooled phosphate buffer, recovered, dissolved in 200 µL of a lysis buffer [20 mM Tris-HCl buffer (pH 7.6) to which 150 mM NaCl, 1 mM MgCl₂, 1% NP 40, 10% glycerol, 8 µL complete protease inhibitor mixture (1 tablet/ml H₂O), 1 mM sodium orthovanadate and 10 mM NaF were added] and allowed to stand for 30 minutes on ice and a supernatant liquid was recovered by centrifugation to carry out extraction of protein.

The extracted protein was fractionated by SDS-PAGE and transferred to a membrane according to a common method and its reactivity with various kinds of antibodies was examined by a western blotting (Antibodies: A Laboratory Manual, Cold Spring Harbor Laboratory, Chapter 12, 1988).

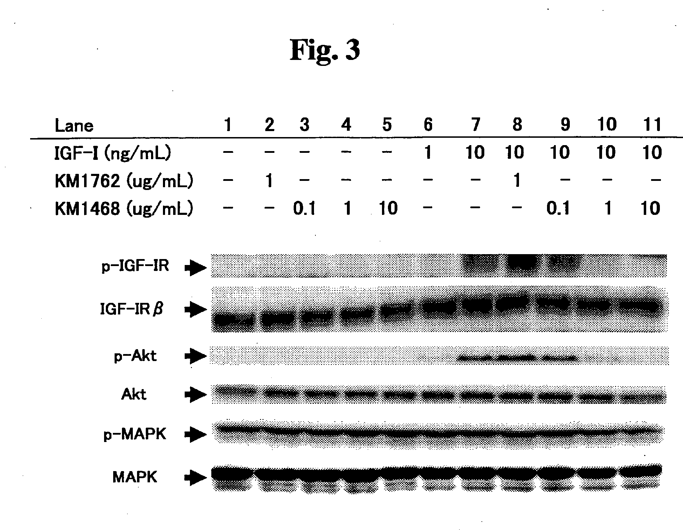

As an antibody recognizing phosphorylated IGF-IR, Akt or MAPK, anti-phosphorylated IGF-IR polyclonal antibody (manufactured by BioSource International), anti-phosphorylated Akt antibody (manufactured by Cell Signaling Technology) or anti-phosphorylated MAPK antibody (manufactured by Cell Signaling Technology) was used respectively while, as an antibody recognizing IGF-IR, Akt or MAPK regardless of phosphorylated and non-phosphorylated, anti-IGF-IRβ antibody (manufactured by Santa Cruz Biotechnology), anti-Akt antibody (manufactured by Cell Signaling Technology) or anti-MAPK antibody (manufactured by Cell Signaling Technology) was used respectively. The result is shown in Fig. 3.

As shown in Fig. 3, phosphorylation of hIGF-IR and Akt was increased by stimulation of hIGF-I (lanes 1, 6 and 7 of Fig. 3). On the other hand, no change in expression of hIGF-IR, Akt, phosphorylated MAPK and MAPK was noted.

The above shows that stimulation by hIGF-I does not transmit a cell proliferation signal mediated by MAPK but transmits a cell survival signal mediated by phosphorylation of Akt from phosphorylated IGF-IR.

### (2) Investigation of inhibitory effect of the antibody KM 1468 to stimulation by hIGF-I

Inhibitory effect of the antibody KM 1468 to the stimulation by hIGF-I was examined by a western blotting mentioned in the above (1). The antibody KM 1468 or KM 1762 (anti-avermectin antibody) which is a negative control was added before addition of hIGF-I under a serum-free condition. The antibody KM 1762 was added at a concentration of 1 µg/mL while the antibody KM 1468 was added at a concentration of 0.1, 1 or 10 µg/mL. The result is shown in Fig. 3.

It was confirmed that, as shown in Fig. 3, phosphorylation was not suppressed in the antibody KM 1762 (lane 8, Fig. 3) while, in the antibody KM 1468, phosphorylation of hIGF-IR and Akt by stimulation of hIGF-1 could be suppressed in a dose dependent manner (lanes 9 to 11, Fig. 3).

Similarly, RPMI 8226 cell were used and an inhibitory action for proliferation of the antibody KM 1468 to cell proliferation by stimulation of hIGF-I was measured by measuring of the number of living cell using trypan blue staining.

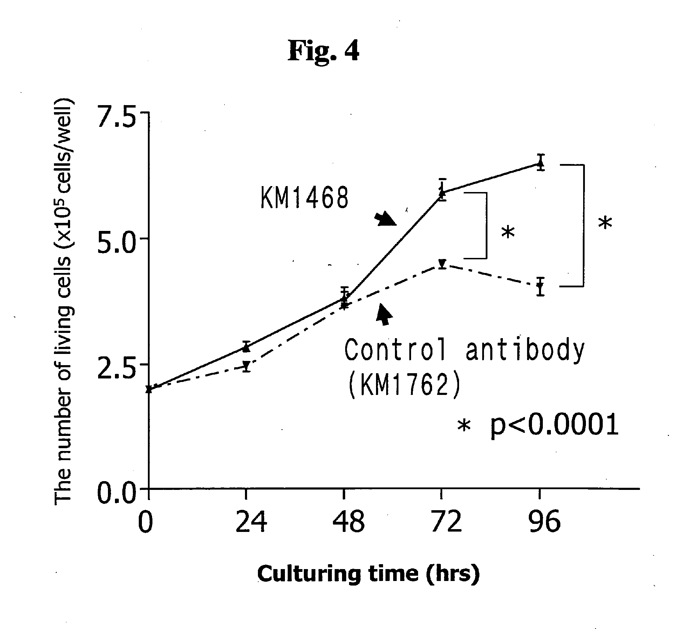

RPMI 8226 cell incubated in a serum-free medium RPMI 1640 (manufactured by Gibco) were inoculated on a 6-well plate at the cell density of 2 × 10⁵ cells per well with or without hIGF-I (manufactured by R & D) in an amount of 1 ng/mL concentration to each well followed by adding the negative control antibody KM 1762 or antibody KM 1468 at a concentration of 1 µg/mL. A stationary culture was carried out in a 5% CO₂ incubator at 37°C, the number of living cell were measured every 24 hours and measurement was carried out until 96 hours thereafter. The result is shown in Fig. 4.

As shown in Fig. 4, it was significantly (p<0.0001) shown that, after 72 hours and 96 hours, proliferation of the cells stimulated by hIGF-I was suppressed by the antibody KM 1468.

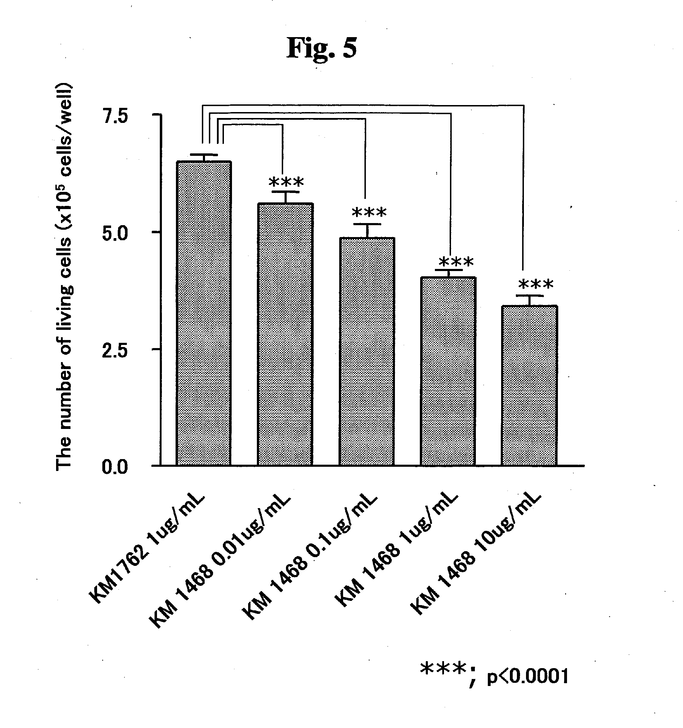

Further, in the same experiment, concentration of the antibody KM 1468 was changed in a stepwise and the result of the measurement of the number of living cell after a stationary culture for 96 hours is shown in Fig. 5.

As shown in Fig. 5, it was confirmed that proliferation of RPMI 8226 cell was suppressed in a dose dependent manner of the antibody KM 1468.

From the above, it was shown that the antibody KM 1468 can suppress the proliferation of multiple myeroma cell line RPMI 8226 cell showing an hIGF-I-dependent proliferation by inhibiting cell survival signal mediated by phosphorylation of Akt from hIGF-IR by stimulation of hIGF-I.

### (3) Examination of inhibitory effect for bone metastasis of multiple osteosarcoma cell line by the antibody KM 1468

Tumor-forming model in human bone was prepared using human multiple osteosarcoma cell line RPMI 8226 cell and examination of suppressive effect of the antibody KM 1468 to tumor formation in bone was carried out.

Human bone tissue piece of about 1 cm³ prepared according to a method of Yonou, et al. (*Cancer Research,* 61, 2177-2182, 2001) was grafted subcutaneously to 6 to 8 weeks-old NOD/SCID mice (Nippon Claire). After 4 weeks from the grafting of the human bone tissue piece, human multiple myeloma cell line RPMI 8226 cell were suspended in a phosphate buffer at the density of 1 × 10⁷ cell/mL and 50 µL of the cell suspension was injected into the grafted human bone tissue piece. Further, at the same time with the grafting of human multiple myeloma cell line RPMI 8226 cell, administration of the antibody KM 1468 was started.

In order to examine the effect of the antibody, the mice were classified into a mouse group to which the antibody KM 1468 was administered (hereinafter, referred to as a treated group) and a mouse group to which an antibody KM 1762 was administered as a negative control (hereinafter, referred to as a control treated group) and administration of the antibody to the mice of each group intraperitoneally was carried out once a week for four weeks. Dose of the antibody KM 1468 per administration in the treated group was 0.1 or 1 mg/kg. Dose of the antibody KM 1762 per administration in the control treated group was 0.1 mg/kg. With regard to the numbers for the test, 10 mice were used in the treated group while, in a control treated group, 5 mice were used. The grafted bone tissues were excised after 4 weeks from the start of the administration and the examination of the effect by administration of the antibody KM 1468 was carried out by the following method.

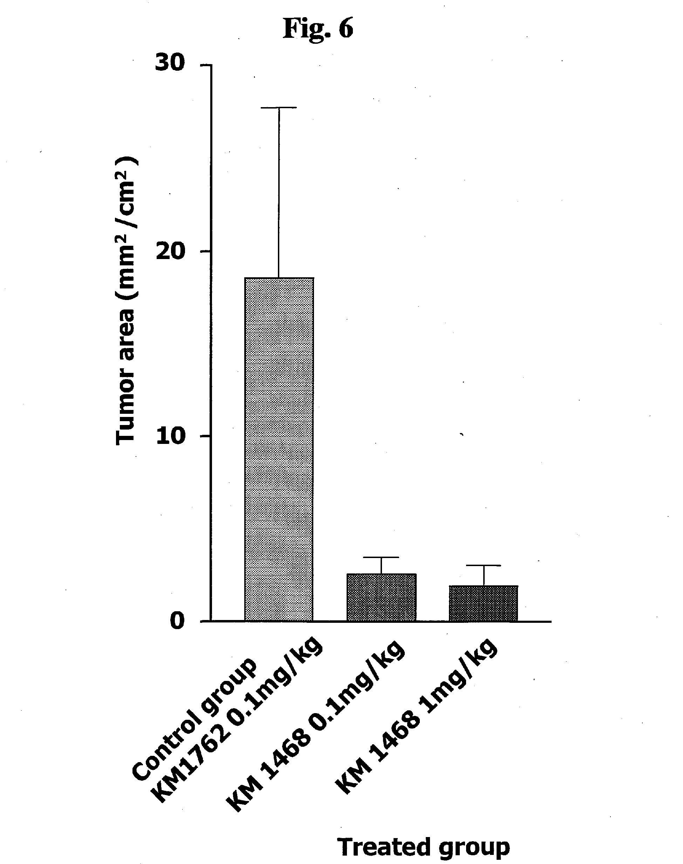

The excised human bone tissue was made into slices and the area occupied by tumor cell in the bone tissue was analyzed using an image analyzer KS 300 system, version 3.00 (manufactured by Carl Zeiss). The result is shown in Fig. 6.

As shown in Fig. 6, mean value of the tumor area in the human bone tissue was 18.52 ± 9.173 mm²/cm² in the control treated group while, in the treated group, it was 2.530 ± 0.9064 mm²/cm² (p = 0.0260) when the antibody KM 1468 was administered at a concentration of 0.1 mg/kg and was 1.890 ± 1.098 mm²/cm² (p = 0.0226) when the antibody KM 1468 was administered at a concentration of 1 mg/kg whereupon a decrease in tumor area was noted in each case as compared with the control treated group.

From the above, it was shown that the antibody KM 1468 can inhibit the adhesion and the proliferation of human multiple myeloma cell line RPMI 8226 cell in the bone tissue transplanted into the body of mouse and can inhibit the hIGF-I-dependent bone metastasis of multiple myeloma. (Example 3)Examination of therapeutic effect of the antibody KM 1468 for bone metastasis of breast cancer cell line

A therapeutic effect of anti-hIGF antibody KM 1468 on bone metastatic focus of breast cancer was examined using a human bone metastatic model of human breast cancer cell line MCF 7 (ATCC HTB-22) cell. As shown in (3) of Reference Example 2, human breast cancer cell line MCF 7 cell is a cell line showing an hIGF-dependent proliferation.

Human bone tissue piece of about 400 cm³ prepared according to a method of Yonou, et al. (*Cancer Research*, 61, 2177-2182, 2001) was grafted subcutaneously to NOD/SCID mice of 6 to 8-weeks old (Nippon Claire). After 4 weeks from the grafting of the human bone tissue piece, human breast cancer cell line MCF 7 cell were suspended in a serum-free medium at a density of 1 × 10⁸ cell/mL and 50 µL of the cell suspension was injected into the grafted human bone tissue piece. Further, administration of the antibody was started simultaneously with the grafting of human breast cancer cell line MCF 7 cell.

The mice were classified into a mouse group to which the antibody KM 1468 was administered (hereinafter, referred to as a treated group) and a mouse group to which an antibody KM 1762 was administered as a negative control (hereinafter, referred to as a control treated group) and administration of the antibody to the mice of each group intraperitoneally was carried out by once a week for four weeks. Dose of the antibody in the treated group and the control treated group per administration was 2 mg/kg. 12 mice were subjected to each of the treated group and the control treated group. In any of the groups, the grafted bone tissues were excised after 4 weeks from the start of the administration.

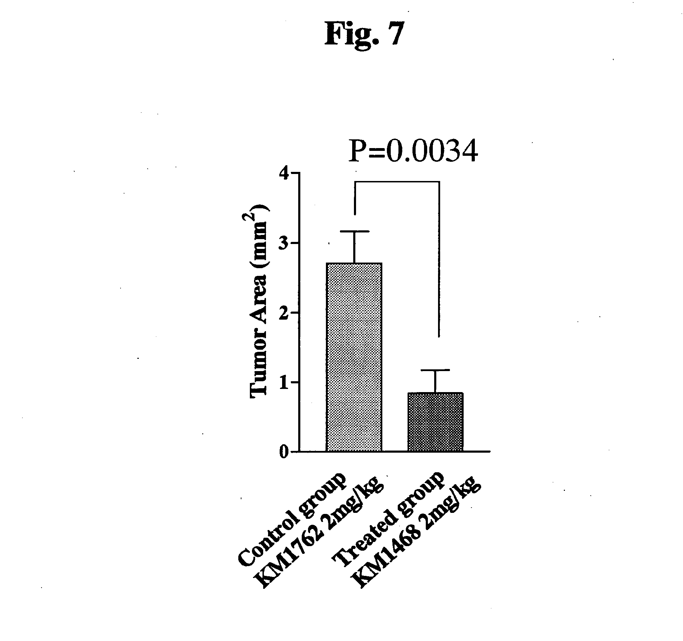

The excised human bone tissue was made into slices and the area occupied by tumor cell in the bone tissue was analyzed using an image analyzer KS 300 system, version 2.00 (manufactured by Carl Zeiss). The result is shown in Fig. 7.

As shown in Fig. 7, the tumor area in the human bone tissue was 2.70 mm² in average in the control treated group while, in the treated group, it decreased to 0.85 mm² in average and to about 30% as compared with the control treated group. From the above, it was shown that the antibody KM 1468 can inhibit the adhesion and the proliferation of human breast cancer cell line MCF 7 cell in the bone tissue grafted into the body of mouse and can inhibit the hIGF-I-dependent bone metastasis of breast cancer.

### (Example 4) Inhibitory effect of antibody mIGF-I antibody and anti-mIGF-II antibody for liver metastasis of colorectal cancer

In order to confirm the inhibitory effect of the anti-mIGF antibody for liver metastasis of human colorectal cancer cell strain HT 29 cells (ATCC HTB-38), the following experiment was carried out using mice.

HT-29 cell were suspended in PBS at a density of 2 × 10⁷ cells/mL and 200 µL of the suspension was injected subcutaneously into a membrane of spleen of male NOD/SCID mice (Nippon Claire) of 9 to 10-weeks old for grafting. Together with the grafting of the HT-29 cell, the mice were classified into the following 5 groups comprising variously treated groups and non-treated group and administration of antibody and PBS was carried out. Further, the number of simultaneous reproducibility of each groups were shown.
1. Non-treated group (administered with PBS); 10 mice
2. Group to which anti-mIGF-I antibody (AF 791 manufactured by R & D) was administered alone; 8 mice
3. Group to which anti-mIGF-II antibody (AF 792 manufactured by R & D) was administered alone; 6 mice
4. Group to which anti-mIGF-I antibody (AF 791 manufactured by R & D) and anti-mIGF-II antibody (AF 792 manufactured by R & D) were co-administered; 7 mice
5. Group to which anti-mIGF-I antibody (AF 791 manufactured by R & D) and the antibody KM 1468 were co-administered; 7 mice

Administration was started on the same day as the grafting of the HT-29 cell and applied intraperitoneally once a week for 4 weeks. Dose for one administration in the treated group was to be 2.5 µg for each antibody per head and, to the non-treated group, PBS at the same volume was administered. Since HT 29 cells secrete a carcinoembryonic antigen (hereinafter, referred to as CEA) which is a tumor marker, CEA value in the mouse serum was measured using an Architect™ CEA kit (manufactured by Dainabbott) and used as an index for proliferation of tumor cell in liver.

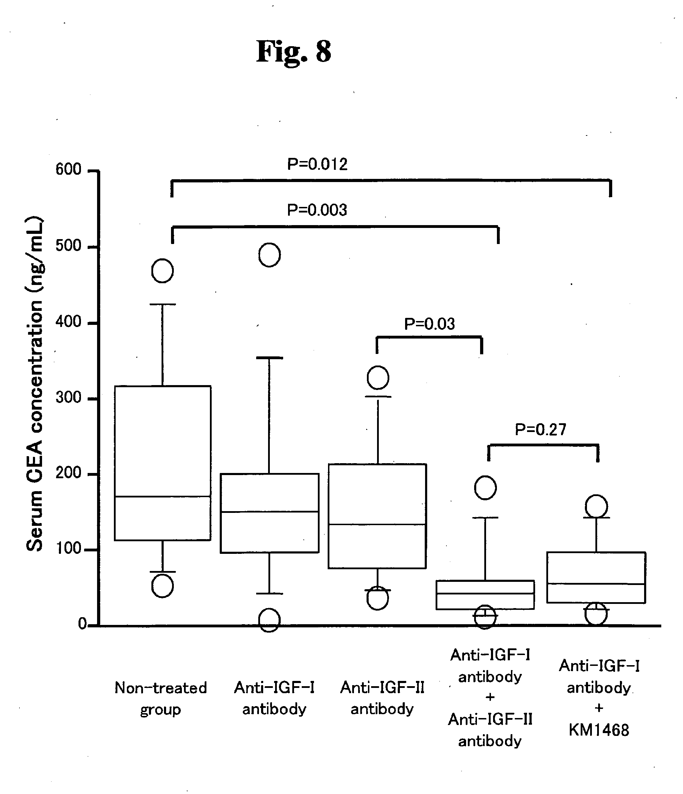

Fig. 8 shows the CEA value in the mouse serum on the 28th day after grafting of the tumor. As compared with the non-treated group, each of the groups to which anti-mIGF-I antibody or anti-mIGF-II antibody was administered alone partially suppressed the proliferation of tumor. On the other hand, in the group where the anti-mIGF-I antibody and the anti-mIGF-II antibody were co-administered and in the group where the anti-mIGF-I antibody and the antibody KM 1486 were co-administered, a significant suppressive activity for proliferation of tumor was noted as compared with the groups where each antibody was administered alone. The antibody KM 1468 is an antibody which neutralizes human IGF-I and IGF-II but, since it shows an activity of selectively neutralizing the IGF-II to the mouse IGF family, it is considered that the antibody KM 1468 functions as a neutralizing antibody to mIGF-II . From the above, it has been made clear that both IGF-I and IGF-II participate in the metastasis of tumor cells and that, for the suppression of tumor, it is effective to simultaneously inhibit the activities of both IGF-I and IGF-II.

Further, in order to analyze the mechanism of suppressive activity for tumor proliferation in the group to which the anti-mIGF-I antibody and the antibody KM 1468 are co-administered, tumor slices were prepared from metastatic focus of mice liver on the 28th day after grafting of tumor for the above-mentioned non-treated group and the group to which the anti-mIGF-I antibody and the antibody KM 1468 were co-administered and were subjected to an immunohistochemical examination. After the liver was excised, it was fixed with 30% formalin and embedded in paraffin to prepare a slice of 5 µm thickness. The slice was subjected to an H&E staining (How to Prepare Pathological Samples (in Japanese), edited by Byori Gijutsu Kenkyukai, 1992)) and stained with anti-cleaved caspase-3 antigen (manufactured by Cell Signaling Technology; used after diluting to 200-fold) using an Envision system (manufactured by DAKO). In the detection, the slice was subjected to a microwave treatment (at 95°C for 20 minutes) in a citrate buffer (pH 6.0) to activate the antigen and used. As a result, in the group where the anti-mIGF-I antibody and the antibody KM 1468 were co-administered, many apoptotic bodies were observed in the H&E staining as compared with the non-treated group and an increase in apoptotic cells stained with the anti-cleave caspase-3 antibody was also noted.

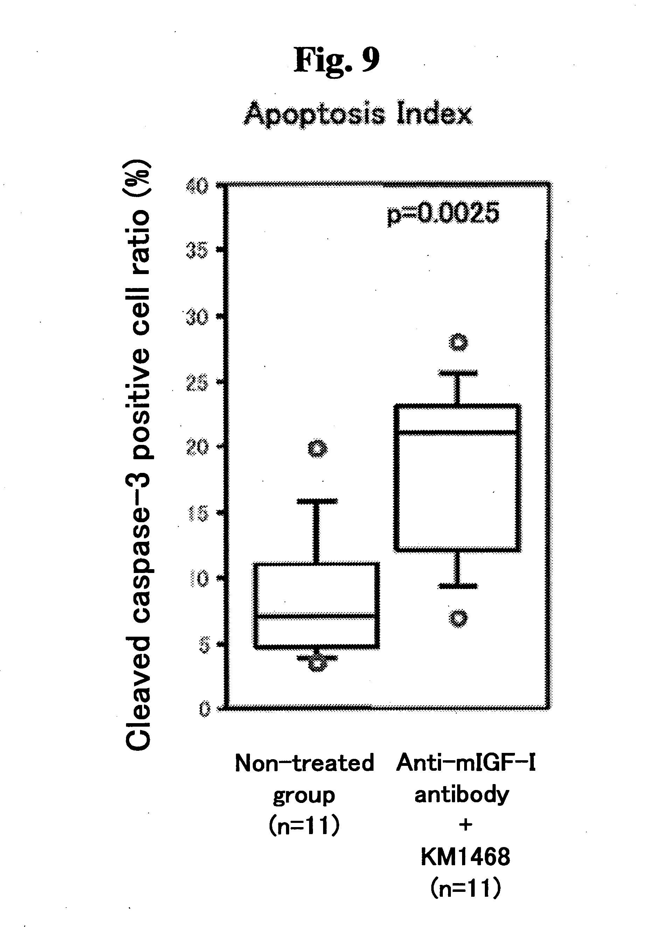

With regard to an index for induction of apoptosis (apoptosis index : AI), the rate (%) of the cleaved caspase-3-positive cells in 1,000 tumor cells were determined and the result is shown in Fig. 9 by graph. As compared with the non-treated group, a significant increase in apoptotic cells was noted in the treated group. From the above result, it has been made clear that, when activities of both IGF-I and IGF-II are inhibited, apoptosis of tumor cell is induced and proliferation of tumor is suppressed.

### (Example 5) Therapeutic effect of anti-mIGF-I antibody and anti-mIGF-II antibody for liver metastatic focus of colorectal cancer

In order to confirm the therapeutic effect of the anti-mIGF antibody to liver metastatic focus of the human colorectal cancer cell line HT-29 cell (ATCC HTB-38), the following experiment was carried out using mice.

HT-29 cell were suspended in PBS at a density of 2 × 10⁷ cells/mL and 200 µL of the suspension was injected subcutaneously into a membrane of spleen of male NOD/SCID mice (Nippon Claire) of 9 to 10-weeks old for grafting. After two weeks from the grafting of the HT-29 cell and metastasis was established in the liver, the mice were classified into the following treated group and non-treated group and administration of antibody and PBS was carried out. Further, the number of simultaneous reproducibility of each groups were shown.
1. Non-treated group (administered with PBS); 8 mice
2. Treated group (a group to which anti-mIGF-I antibody and antibody MK 1468 were co-administered); 9 mice

Administration of antibody was started after two weeks from the grafting of HT-29 cell and applied intraperitoneally once a week for 4 weeks. Dose for one administration in the treated group was 0.1 µg for anti-mIGF-I antibody (AF 791 manufactured by R & D) and 1 µg for the antibody KM 1468 per kg of the body weight of the mouse and, to the non-treated group, PBS in the same volume was administered. CEA value in the mouse serum was measured using an Architect™ CEA kit (manufactured by Dainabbott) and used as an index for proliferation of tumor cell in liver.

In Fig. 10A, CEA values in the mouse serum on the 28th day after the administration was started are shown. As compared with the non-treated group, a significant suppressive effect for proliferation of tumor was noted in the treated group. Fig. 10B shows an accumulated survival rate of the mouse is shown. On the 41st day after the administration started, all mice died in the non-treated group while, in the treated group, a significant survival effect was confirmed and, on the 42nd day where the experiment finished, 4 mice were still alive. From the above, it has been made clear that simultaneously inhibition of activities of both IGF-I and IGF-II shows an effective therapeutic effect for liver metastatic focus of colorectal cancer and that a therapeutic method where activities of both IGF-I and IGF-II are simultaneously inhibited has a survival effect.

### (Reference Example 1) Preparation of anti-hIGF monoclonal antibody

### (1) Immunization of animal and preparation of antibody-producing cell

A recombinant hIGF-I (manufactured by R & D) was made into a conjugate with a methylated BSA (manufactured by Sigma) for the purpose of increasing its immunogenicity, and use as the immunogen. Thus, methylated BSA dissolved in redistilled water was mixed at 4°C so as to make methylated BSA:hIGF = 1:4 (ratio by weight) and stirred for 10 seconds in a vortex mixer. After that, it was mixed with complete Freund's adjuvant or incomplete Freund's adjuvant using a syringe equipped with a connecting needle at a ratio by volume of 1:1 to give an immunogen (hereinafter, referred to as methylated BSA-hIGF-I).

The methylated BSA-hIGF-I (equivalent to 100 µg of hIGF-I) prepared as above using a complete Freund's adjuvant was administered to a female SD rat of 5-weeks old and, from two weeks thereafter, an immunogen which was similarly prepared using an incomplete Freund's adjuvant was administered once a week for 4 times in total.

Blood was collected from venous plexus of the fundus of the eye, antibody titer in its serum was checked by a binding ELISA shown in Reference Example 1(4) and spleen was excised from a rat showing a sufficient antibody titer after 3 days from the final immunization.

After the spleen was cut into pieces in an MEM medium (manufactured by Nissui Seiyaku), loosened by tweezers and centrifuged (at 1,200 rpm for 5 minutes), the supernatant was discarded, the resulting precipitate was treated with a Tris-ammonium chloride buffer (pH 7.65) for 1 to 2 minutes to eliminate erythrocytes, and the remainder was washed with MEM for 3 times to be used for cell fusion.

### (2) Preparation of mouse myeloma cells

An 8-azaguanine-resistant mouse myeloma cell line P3-U1 was incubated in a common medium and not less than 2 × 10⁷ cells were secured upon cell fusion to be used as a parent cell for cell fusion.

### (3) Preparation of hybridoma

The rat spleen cell prepared in Reference Example 1(1) and the myeloma cell prepared in (2) were mixed so as to make their ratio 10:1 followed by centrifuging (at 1,200 rpm for 5 minutes), the supernatant was discarded, 0.2 to 1.0 mL of a fusion medium (a mixture of 2 g of PEG 1000, 2 mL of MEM and 0.7 mL of dimethyl sulfoxide) per 10² rat spleen cell was added to the precipitated cell with stirring at 37°C, 1 to 2 mL of MEM was added for several times every 1 to 2 minutes and MEM was further added thereto so that the total volume was made 50 mL. After centrifugation (at 900 rpm for 5 minutes), the supernatant was discarded and the resulting cell were gently loosened and suspended in 100 mL of HAT medium.

The suspension was dispensed in a 96-well plate for incubation in an amount of 100 µL/well and incubated in a 5% CO₂ incubator for 10 to 14 days at 37°C. The culture supernatant was subjected to a binding ELISA shown in Reference Example 1(4) to select wells which reacted with methylated BSA-hIGF-I and did not react with methylated BSA-BSA which is a negative control [a conjugate prepared by the same reaction as in the above Referential Example 1(1) using BSA], and anti-hIGF-I rat monoclonal antibody-producing hybridoma were established by carrying out single cell cloning twice by changing the medium to HT medium and the normal medium.

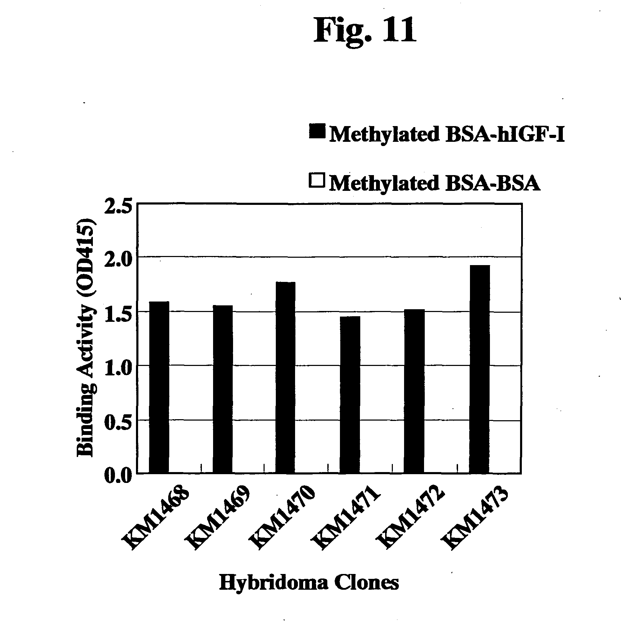

As a result, 6 hybridoma clones of KM 1468, KM 1469, KM 1470, KM 1471, KM 1472 and KM 1473 having reactivities shown in Fig. 11 were obtained. When subclass of the antibody produced by each hybridoma was examined by an ELISA using a subclass typing kit, all of the subclasses were IgG2b.

### (4) Selection of monoclonal antibody (combined ELISA)

As to an antigen to be immobilized to an ELISA plate, the methylated BSA-hIGF-I prepared in Reference Example 1(1) was used while, as to a negative control, methylated BSA-BSA was used. The above antigen in 10 µg/mL in terms of concentration of hIGF-I or BSA was dispensed in a 96-well ELISA plate (manufactured by Greiner) in an amount of 50 µL/well and allowed to stand over night at 4°C for immobilized. After washing with PBS, PBS containing 1% of BSA (hereinafter, referred to as BSA-PBS) was added in an amount of 100 µL/well and reacted at room temperature for 1 hour to block the remaining active group. The BSA-PBS was discarded and then rat antiserum to be immunized, culture supernatant of hybridoma which produces anti-hIGF-I monoclonal antibody or purified anti-hIGF-I monoclonal antibody was dispensed in an amount of 50 µL/well and reacted at room temperature for 2 hours. After the reaction, each well was washed with PBS containing 0.05% of Tween 20 (hereinafter, referred to as Tween-PBS) and 50 µL/well of peroxidase-labeled rabbit anti-rat Ig antibody diluted to 4,000-fold (manufactured by Dako) was added as a secondary antibody and allow to react at room temperature for 1 hour. After the reaction, it was washed with Tween-PBS, 50 µL/well of ABTS substrate solution [a solution prepared by dissolving 0.55 g of ammonium 2,2'-azino-bis(3-ethylbenzothiazoline-6-sulfonate) in 1L of 0.1 M citrate buffer (pH 4.2) followed by adding 1 µL/ml of an aqueous solution of hydrogen peroxide immediately before use] was then added thereto to effect color development and absorbance at 415 nm (hereinafter, referred to as OD415) was measured using a plate reader Emax (manufactured by Molecular Devices).

### (5) Purification of monoclonal antibody

The hybridoma clone prepared in Reference Example 1(3) was intraperitoneally injected in an amount of 5 to 20 × 10⁶ cells/mouse into pristane-treated 8-weeks old female Balb/c nude mice. After 10 to 21 days, ascites was collected (1 to 8 mL/mouse) from the mice where the hybridoma,turned ascites cancer and centrifuged (at 3,000 rpm for 5 minutes) to remove solids. After that, IgG fraction was purified by a caprylic acid precipitation method (Antibodies, A Laboratory Manual, Cold Spring Harbor Laboratory, 1988) to give a purified monoclonal antibody.

### (Reference Example 2) Examination of reactivity of anti-hIGF monoclonal antibody

### (1) Reactivity of hIGF-I to natural three-dimensional structure

Reactivity of the anti-hIGF monoclonal antibody selected in Referentce Example 1(3) to hIGF-I maintaining natural three-dimensional structure in a liquid phase system was examined by the following competitive ELISA.

A plate where the methylated BSA-hIGF-I prepared in Reference Example 1(1) was immobilized as shown in Reference Example 1(4) was prepared, hIGF-I which was diluted in 5-fold serial dilutions from 20 µg/mL was dispensed in an amount of 50 µL/well, then a solution where the purified antibody of the anti-hIGF monoclonal antibody was diluted (KM 1468: 6.0 µg/mL, KM 1470: 1.0 µg/mL, KM 1471: 0.16 µg/mL, KM 1472: 7.0 µg/mL, KM 1473: 1.2 µg/mL) was dispensed in an amount of 50 µL/well followed by mixing and the mixture was allowed to react at room temperature for 2 hours. After the reaction, it was washed with Tween-PBS and then 50 µL/well of peroxidase-labeled rabbit anti-rat Ig antibody (manufactured by Dako) diluted to 4,000-fold was added followed by reacting at room temperature for 1 hour. After the reaction, it was washed with Tween-PBS, 50 µL/well of an ABTS substrate solution [a solution prepared by dissolving 0.55 g of ammonium 2,2'-azino-bis(3-ethylbenzothiazoline-6-sulfonate) in 1L of 0.1 M citrate buffer (pH 4.2) followed by adding with 1 µL/ml of an aqueous solution of hydrogen peroxide immediately before use] was added thereto to effect color development and OD415 was measured using a plate reader Emax (manufactured by Molecular Devices).

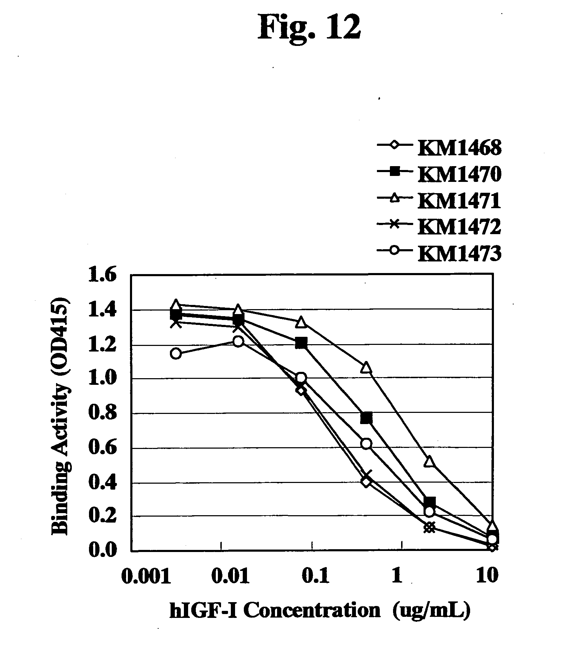

As shown in Fig. 12, all of the six anti-hIGF monoclonal antibodies of the present invention showed reactivity to a natural three-dimensional structure of hIGF-I. In addition, when KM 1468 showing the highest sensitivity in the present system was used, hIGF-I having a natural three-dimensional structure contained in the liquid phase system can be detected up to a concentration of 16 ng/mL.

### (2) Reactivity of the anti-hIGF monoclonal antibody to hIGF family

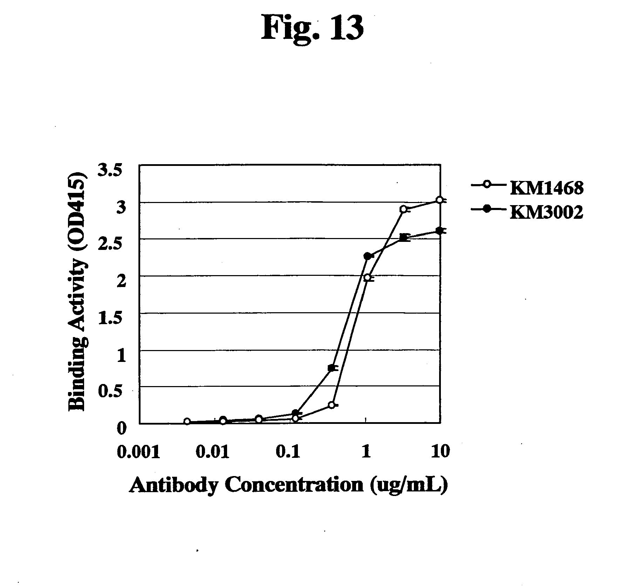

Reactivity of the purified anti-hIGF monoclonal antibody KM 1468 (hereinafter, referred to as antibody MK 1468) to hIGF was examined. Fig. 13 shows the result of the examinnation on the reactivity of the antibody KM 1468 and sm1.2 (manufactured by Upstate Biotechnology) which is a commercially available anti-hIGF-I antibody to hIGF-I by binding ELISA shown in Reference Example 1(4) (in which antibody concentration was diluted in a 3-fold serial dilutions from 30 µg/mL). In the case of sm1.2 however, a peroxidase-labeled rabbit anti-mouse Ig antibody (manufactured by Dako) diluted to 2,000-fold was used as a secondary antibody. Although any of the antibodies showed a hIGF-I-binding activity dependent upon concentration of the antibody as shown in Fig. 13, the activity is higher in the case of the antibody KM 1468.

Then an inhibitory activity by hIGF-I (manufactured by Pepro Tech EC), hIGF-II (manufactured by Pepro Tech EC), human insulin (manufactured by Wako Pure Chemical) and mIGF-I (manufactured by Pepro Tech EC) in a binding of each antibody to hIGF-I was examined by the following competitive ELISA.

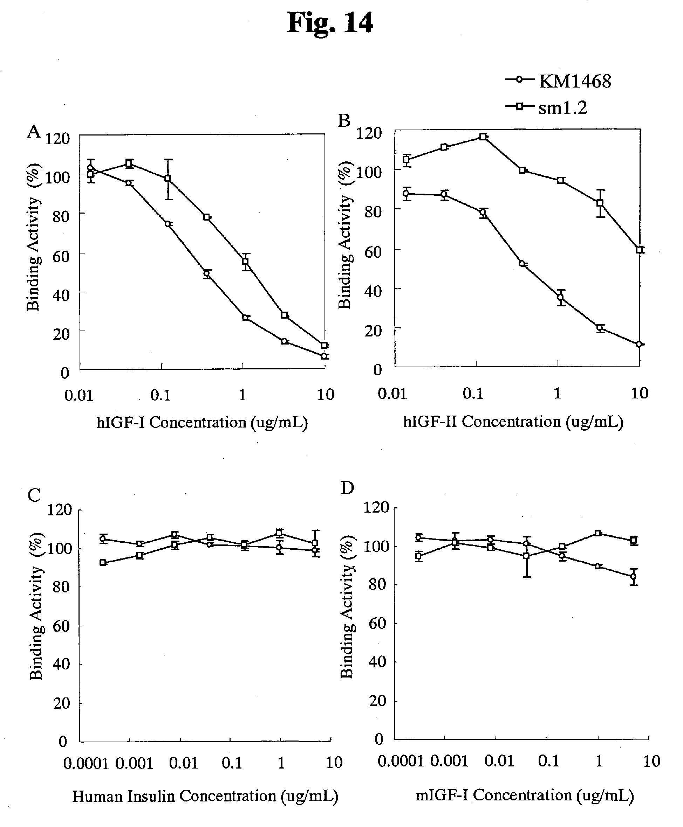

As shown in Reference Example 1(4), a plate where antigen was immobilized was prepared, each antibody diluted to 4.0 µg/mL was dispensed in an amount of 50 µL/well, then hIGF-I or hIGF-II diluted in a 3-fold dilution step from 20 µg/mL or human insulin or mIGF-I diluted in a 5-fold serial dilutions from 10 µg/mL was dispensed in an amount of 50 µL/well and they were mixed and reacted at room temperature for 1 hour. After the reaction, it was washed with Tween-PBS and, in the case of KM 1468, a peroxidase-labeled rabbit anti-rat Ig antibody (manufactured by Dako) diluted to 4,000-fold or, in the case of sm1.2, a peroxidase-labeled rabbit anti-rat Ig antibody (manufactured by Dako) diluted to 2,000-fold was added in an amount of 50 µL/well followed by subjecting to a reaction at room temperature for 1 hour. After the reaction, it was washed with Tween-PBS, 50 µL/well of ABTS substrate solution [a solution prepared by dissolving 0.55 g of ammonium 2,2'-azino-bis(3-ethylbenzothiazoline-6-sulfonate) in 1L of 0.1 M citrate buffer (pH 4.2) following by adding with 1 µL/ml of an aqueous solution of hydrogen peroxide immediately before use] was added thereto to effect to develop color and OD415 was measured using a plate reader Emax (manufactured by Molecular Devices). The result was given in terms of a relative value (%) where the OD415 when only antibody was added was defined as 100. The result was shown in Fig. 14. As shown in Fig. 14, binding of the antibody KM 1468 to hIGF-I was strongly inhibited by hIGF-I (Fig. 14A) and hIGF-II (Fig. 14B) and a 50% inhibition concentration (hereinafter, referred to as IC₅₀) for the binding by hIGF-I was about 0.3 µg/mL (about 39 nM) while the IC₅₀ by hIGF-II was about 0.4 µg/mL (about 58 nM) whereby they showed nearly the same value. On the other hand, no inhibition was noted in human insulin and mIGF-I. From the above result, it has been clarified that the antibody KM 1468 reacts with both hIGF-I and hIGF-II almost the same specificity and almost the same degree. Binding of sm1.2 which is the commercially available anti-IGF-I antibody to hIGF-I was strongly inhibited by hIGF-I (Fig. 14A) and an inhibitory activity by hIGF-II (Fig. 14B) was weak. IC₅₀ of sm1.2 by hIGF-I was about 1.2 µg/mL (about 156 nM) while IC₅₀ by hIGF-II was >10 µg/mL (> 1.45 µM). On the other hand, no inhibition was noted in human insulin and mIGF-I.

### (3) Influence of anti-hIGF monoclonal antibody on hIGF-dependent cell proliferation

Influence of purified antibody KM 1468 on hIGF-dependent cell proliferation was examined. With regard to the antibody, KM 1468, sm1.2 (manufactured by Upstate Biotechnology) which is a commercially available anti-hIGF-I antibody and S1F2 (manufactured by Upstate Biotechnology) which is a commercially available anti-hIGF-II antibody were used.

Human breast cancer cell line MCF 7 (ATCC HTB-22), human colorectal cancer cell line HT-29 (ATCC HTB-38) or human osteosarcoma cell line MG-63 (ATCC CRL-1427) was prepared into 0.5 to 1 × 10⁵ cells/mL using a TF/BSA medium [a medium where 10 µg/mL of human transferrin (manufactured by Gibco BRL) and 200 µg/mL of BSA were added to D-MEM/F-12 (manufactured by Gibco BRL)] and dispensed in a 96-well plate for incubation in an amount of 100 µL/well. Subsequently, each factors of hIGF-I, hIGF-II and human insulin diluted to each concentration with the TF/BSA medium was added in amount of 50 µl/well thereto, and each of the antibodies diluted to each concentration with the TF/BSA medium was added in amount of 50 µl/well, and cultured at 37°C for 5 days in a 5% CO₂ incubator. After incubation, a cell proliferation reagent WST-1 (manufactured by Roche) was disposed in an amount of 20 µL/well and incubated for 2.5 to 4 hours in a 5% CO₂ incubator at 37°C and then absorbance at OD450 nm (hereinafter, referred to as OD450) was measured using a plate reader Emax (manufactured by Molecular Devices).

Fig. 15A shows a proliferation curve of human breast cancer cell line MCF 7 by each factor. Further, Fig. 15B shows proliferation upon addition of each antibody in the presence of 40 ng/mL of hIGF-I, Fig. 15C shows the proliferation in the presence of 100 ng/mL of hIGF-II and Fig. 15D shows the proliferation in the presence of 100 ng/mL of human insulin. As shown in Fig: 15, KM 1468 strongly inhibits the cell proliferation by hIGF-I and hIGF-II in the same degree and its activity was higher than sm1.2 which is the commercially available anti-hIGF-I antibody and than S1F2 which is a commercially available anti-hIGF-II antibody. On the other hand, none of antibodies affected the growth by human insulin. The above result clearly shows that there is a good correlation to the binding specificity of each antibody seen in the competitive ELISA of Reference Example 2 (2) and that activity of hIGF-I and hIGF-II is inhibited by binding of each antibody.

Fig. 16A shows a proliferation curve of human colorectal cancer cell line HT-29 by each factor. Fig. 16B shows proliferation upon addition of each antibody in the presence of 10 ng/mL of hIGF-I, Fig. 16C shows the proliferation in the presence of 10 ng/mL of hIGF-II and Fig. 16D shows the proliferation in the presence of 20 ng/mL of human insulin.

As shown in Fig. 16, KM 1468 strongly inhibits the cell proliferation by hIGF-I and hIGF-II in the same degree and its activity was higher than sm1.2 which is the commercially available anti-hIGF-I antibody as well as S1F2 which is a commercially available anti-hIGF-II antibody. On the other hand, none of antibodies affected the proliferation by human insulin. The above result clearly shows that there is a good correlation to the binding specificity seen in the competitive ELISA of Reference Example 2(2) and that activity of hIGF-I and hIGF-II is inhibited by binding of each antibody. Further, when KM 1468 of Fig. 16B and KM 1468 and S1F2 of Fig. 16C were allowed to react, cell proliferation was suppressed as compared with the case where hIGF-I and hIGF-II were not added. From the above, it has been made clear that HT-29 cell are proliferated by production of hIGF-I and hIGF-II by themselves and that the proliferation effect can also be inhibited by addition of antibody.

Fig. 17A shows a proliferation curve of human osteosarcoma cell line MG-63 by each factor. Fig. 17B shows proliferation upon addition of each antibody in the presence of 20 ng/mL of hIGF-I, Fig. 17C shows the proliferation in the presence of 20 ng/mL of hIGF-II and Fig. 17D shows the proliferation in the presence of 20 ng/mL of human insulin. As shown in Fig. 17, KM 1468 strongly inhibits the cell proliferation by hIGF-I and hIGF-II in the same degree and its activity was higher than sm1.2 which is the commercially available anti-hIGF-I antibody as well as S1F2 which is a commercially available anti-hIGF-II antibody. On the other hand, none of antibodies affected the proliferation by human insulin. The above result clearly shows that there is a good correlation to the binding specificity seen in the competitive ELISA of Reference Example 2 (2) and that function of each factor is inhibited by binding of each antibody. (Reference Example 3) Analysis of antigen-recognizing site of anti-hIGF monoclonal antibody

### (1) Synthesis of partial peptide of hIGF-I

A partial peptide of hIGF-I was synthesized according to a method mentioned in WO 01/64754. The synthesized peptide is a peptide corresponding to 1st to 18th (SEQ ID NO: 1; hereinafter, referred to as p1-18), 14th to 30th (SEQ ID NO: 2; hereinafter, referred to as p14-30), 24th to 35th (SEQ ID NO: 3; hereinafter, referred to as p24-35), 29th to 41st (SEQ ID NO: 4; hereinafter, referred to as p29-41), 36th to 47th (SEQ ID NO: 5; hereinafter, referred to as p36-47), 41st to 56th (SEQ ID NO: 6; hereinafter, referred to as p41-56), 52nd to 70th (SEQ ID NO: 7; hereinafter, referred to as p52-70), 53rd to 61st (SEQ ID NO: 8; hereinafter, referred to as p53-61) and 61st to 70th (SEQ ID NO: 9; hereinafter, referred to as p61-70) of hIGF-I and was designed to cover the full length of hIGF-I. In the above-mentioned peptides, a sequence where Cys existing therein was substituted with Ser or Ala was synthesized. With regard to the sequence corresponding to 41st to 56th, a sequence having an inner Cys (SEQ ID NO: 10; hereinafter, referred to as p41-56C) was also synthesized.

### (2) Analysis of antigen-recognizing site of anti-hIGF monoclonal antibody

Analysis of antigen-recognizing site of anti-hIGF rat antibody KM 1468 was examined by the following competitive ELISA using various kinds of peptides synthesized in the above (1).

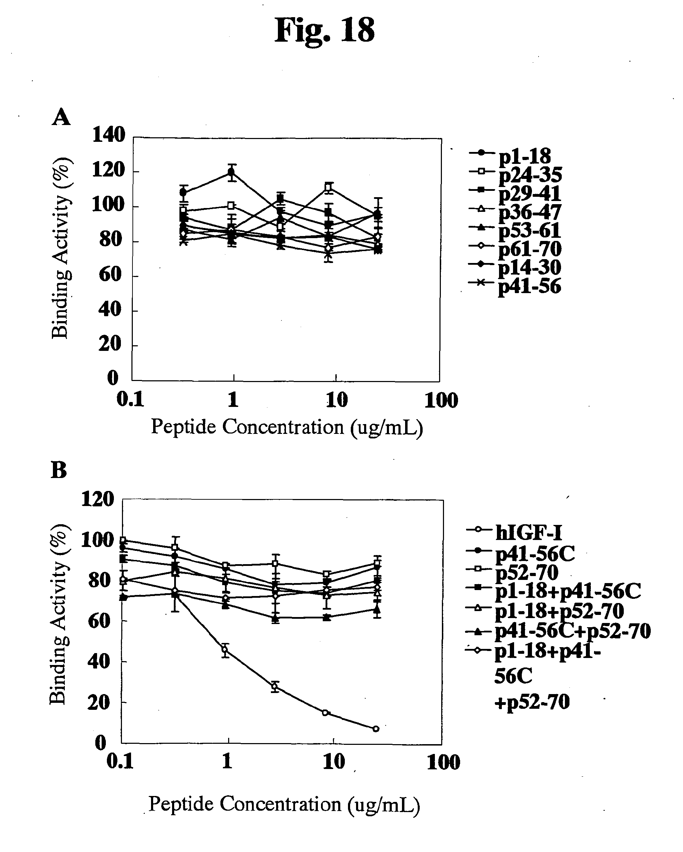

As shown in Reference Example 1(4), a plate where antigen was immobilized was prepared, various antibodies diluted to 4.0 µg/mL were dispensed in 50 µL/well and either alone or various combinations of various peptide solutions diluted in 3-fold serial dilutions from 50 µg/mL or hIGF-I was dispensed in 50 µL/well followed by mixing and subjecting to a reaction at room temperature for 1 hour. After the reaction, the above was washed with Tween-PBS, a peroxidase-labeled rabbit anti-rat Ig antibody (manufactured by Dako) diluted to 4, 000-fold was added in an amount of 50 µL/well and was allowed to react at room temperature for 1 hour. After the reaction, it was washed with Tween-PBS, an ABTS substrate solution [a solution prepared by dissolving 0.55 g of ammonium 2,2'-azino-bis(3-ethylbenzothiazoline-6-sulfonate) in 1L of 0.1M citrate buffer (pH 4.2) followed by adding 1 µL/mL of aqueous hydrogen peroxide solution thereto immediately before use] was added thereto in an amount of 50 µL/well to effect to develop color and OD415 was measured using a plate reader Emax (manufactured by Molecular Devices). The result is shown in terms of a relative value (%) where the OD415 when only antibody was added was defined as 100. The result is shown in Fig. 18. As shown in Fig. 18, binding of KM 1468 to hIGF-I was dose-dependently inhibited by hIGF-I but, in the cases of various peptides, no inhibitory activity was noted regardless of sole or combined use thereof. The above result strongly suggests that KM 1468 is not a mere amino acid primary sequence of hIGF-I but recognizes a three-dimensional structure of hIGF-I.

All publications, patents and patent applications cited in the present specification shall be incorporated as references into the present specification as they are.

### Industrial Applicability

The cancer metastasis inhibitor of the present invention can significantly inhibit metastasis of cancer such as bone metastasis of prostate cancer and liver metastasis of colorectal cancer and, therefore, it is clinically quite useful as a pharmaceutical composition for the treatment of cancer.

In addition, the cancer metastasis inhibitor of the present invention is particularly effective for suppression of proliferation of tumor cell of the metastatic focus and, therefore, it can also be administered for prevention to a patient whose symptom of cancer metastasis is not clearly seen. Further, it can suppress the proliferation of tumor cell of the metastatic focus and, therefore, it can also be effectively used as a pharmaceutical composition for metastatic focus of late state of the disease.

## Claims

1. A cancer metastasis inhibitor comprising a substance which inhibits an activity of insulin-like growth factor-I (IGF-I) and insulin-like growth factor-II (IGF-II) as an active ingredient.

2. The inhibitor according to claim 1, wherein the substance is selected from a group consisting of the following (a) to (e).
(a) an antibody or an antibody fragment which specifically binds to IGF-I and IGF-II and inhibits the activity of IGF-I and IGF-II;
(b) a composition comprising an antibody or an antibody fragment which specifically binds to IGF-I and inhibits the activity of IGF-I and an antibody or an antibody fragment which specifically binds to IGF-II and inhibits the activity of IGF-II;
(c) a composition comprising combination of a composition comprising an antibody or an antibody fragment which specifically binds to IGF-I and inhibits the activity of IGF-I and a composition comprising an antibody or an antibody fragment which specifically binds to IGF-II and inhibits the activity of IGF-II;
(d) a conjugate of an antibody or an antibody fragment which specifically binds to IGF-I and inhibits the activity of IGF-I with an antibody or an antibody fragment which specifically binds to IGF-II and inhibits the activity of IGF-II; and
(e) a conjugate of any of the above (a) to (d) with the other molecules.

3. The inhibitor according to claim 2, wherein the antibody is a monoclonal antibody.

4. The inhibitor according to claim 2 or 3, wherein the antibody fragment is an antibody fragment selected from the group consisting of Fab, Fab' , F(ab')₂, single-chain antibody (scFv), dimerized variable region (Diabody), disulfide-stabilized variable region (dsFv) and CDR-containing peptide.
